# EUROPEAN PATENT APPLICATION

(11) **EP 3 868 755 A1**
(43) Date of publication of application: **25.08.2021**
(21) Application number: 19874641.4
(22) Date of filing: 17.10.2019
(51) Int. Cl.: C07D 405/14, C07D 487/04, A61P 35/00, A61P 35/02, A61P 1/16, A61P 29/00, A61P 13/12, A61K 31/4709, A61K 31/5377, A61K 31/541, A61K 31/4985

(54) **COMPOUND AS TGF-? R1 INHIBITOR AND APPLICATION THEREOF**

(30) Priority: 18.10.2018 CN 201811211890
(71) Applicant: Nanjing Sanhome Pharmaceutical Co., Ltd., Nanjing Jiangsu 210038 (CN)
(72) Inventor: WANG, Yong, Nanjing, Jiangsu 210038 (CN); ZHAO, Liwen, Nanjing, Jiangsu 210038 (CN); WANG, Yazhou, Nanjing, Jiangsu 210038 (CN); ZHANG, Yan, Nanjing, Jiangsu 210038 (CN); WANG, Xiaowei, Nanjing, Jiangsu 210038 (CN); WANG, Hai, Nanjing, Jiangsu 210038 (CN); GUO, Zhuang, Nanjing, Jiangsu 210038 (CN); LV, Kunzhi, Nanjing, Jiangsu 210038 (CN); CHANG, Yujie, Nanjing, Jiangsu 210038 (CN); CHEN, Hongyan, Nanjing, Jiangsu 210038 (CN); XU, Guofeng, Nanjing, Jiangsu 210038 (CN)
(74) Representative: ABG Intellectual Property Law, S.L.
(86) International application number: PCT/CN2019/111568
(87) International publication number: WO 2020/078402

(57) **Abstract**

The present application relates to a compound as a TGF-β R1 inhibitor and application thereof. Specifically, provided are a compound of formula I, or an isomer, pharmaceutically acceptable salt, solvent, crystal, or prodrug thereof, preparation methods therefor, and pharmaceutical compositions comprising these compounds, and applications of these compounds or compositions in treating and/or preventing TGF-β R1-related diseases, such as cancer, tissue proliferation diseases, fibrosis, and inflammatory diseases. The compound represents a significant inhibitory activity for TGF-β R1 kinase, and is promising as a treatment agent for TGF-β R1-related diseases.

## Description

### FIELD

The present disclosure belongs to the field of medical chemistry, and specifically relates to a group of compounds of TGF-βR1 inhibitors or pharmaceutically acceptable salts, isomers, solvates, crystals or prodrugs thereof, their preparation methods, and the application of the pharmaceutical composition containing these compounds, these compounds or compositions in treating and/or preventing the diseases related to TGF-βR1 expression, such as cancer and myelodysplatic syndrome.

### BACKGROUND

TGF-β (transforming growth factor beta) is a group of important cytokines. So far, it has been found that they includes 6 different subtypes (TGF-β 1 to 6) having homology different from each other, and only 3 isoforms are expressed in mammals, namely TGF-β1, TGF-β2 and TGF-β3. It is a multifunctional growth factor superfamily, having a wide range of biological activities, involving in early embryonic development, the formation of cartilage and bone, the synthesis of extracellular matrix, inflammation, interstitial fibrosis, the regulation of immunity and endocrine function, and the formation and development of tumors. Meanwhile, these three subtypes are similar in structures, and their amino acid sequences have high homology, but in the gene knockout mouse models, each of them exhibits totally different phenotypes, suggesting that each subtype has a specific and non-intersecting functions in the body. TGF-β family ligands all can bind to receptors on the membrane surface, to initiate the downstream signal transmission in a cell.

TGF-β1 is the most common and important isoform of TGFβ, an isoform expressed mostly abundantly in the liver and the strongest liver fibrosis-inducing factor known, which plays an important role in the development from chronic liver disease to end-stage liver disease (Yamazaki,et al. Digestive Disease, 2011, 29 : 284-288). Many studies have demonstrated that TGF-β1 and TGFβ receptors are usually highly expressed in diseased liver organs, blood vessels and the extracellular matrix. In the typical TGFβ-TGFβR-Smads pathway, TGF-β1 activates TGFβR1 (transforming growth factor beta receptor 1, ALK5) in the signaling pathway, and then regulates the entire signaling pathway, to achieve the regulation of the expression of target genes related to the occurrence and development of fibrosis and tumors. It is generally believed that the promoting effects of TGF-β on liver cancer are mainly manifested in the aspects of promoting tumor cell metastasis, enhancing tumor cells to escape from immunosurveillance and inducing angiogenesis (Ling, et al. Current Pharmaceutical Biotechnology, 2011, 12 : 2190-2202).

Research on drugs targeting the TGF-β pathway has been conducted for many years, but TGFβR1 inhibitors such as Galunisertib show certain cardiotoxicity (such as bleeding, functional deterioration, and inflammatory injury) in animal models, which is attributed to the low target-site selectivity and specificity of these drugs, i.e., while the drugs inhibit the activation site of TGFβR1 kinase, they also have a strong inhibitory effect on other proteins with the same kinase domain (such as p38α), which further produces many unexpected off-target toxic side effects. Therefore, there is still a need to develop more selective TGFβR1 inhibitors in order to specifically regulate the TGF-β signaling pathway for the treatment of TGF-β-related diseases.

### SUMMARY

An object of the present disclosure is to provide a group of compounds represented by following general formula I having TGF-βR1 inhibitory activity or pharmaceutically acceptable salts, isomers, solvates, crystals or prodrugs thereof,

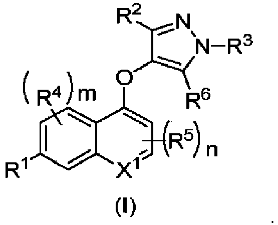

Another object of the present disclosure is to provide a method of preparing the compounds of general formula I or isomers, pharmaceutically acceptable salts, solvates, crystals, isosteres or prodrugs thereof of the present disclosure.

Yet another object of the present disclosure is to provide a composition comprising a compound of general formula I or an isomer, pharmaceutically acceptable salt, solvate, crystal, isostere or prodrug thereof of the present disclosure and a pharmaceutically acceptable carrier, and a composition comprising a compound of general formula I or an isomer, pharmaceutically acceptable salt, solvate, crystal, isostere or prodrug thereof of the present disclosure and another one or more drugs.

Further another object of the present disclosure is to provide a method of treating and/or preventing the disease related to TGF-βR1 by using a compound of general formula I or an isomer, pharmaceutically acceptable salt, solvate, crystal, isostere or prodrug thereof of the present disclosure, and the use of a compound of general formula I or an isomer, pharmaceutically acceptable salt, solvate, crystal, isostere or prodrug thereof of the present disclosure in the manufacture of a medicament for treating and/or preventing a disease related to TGF-βR1.

For the purposes described above, the present disclosure provides the following technical solutions.

In one aspect, the present disclosure provides a compound represented by general formula I or an isomer, pharmaceutically acceptable salt, solvate, crystal, isosteres or prodrug thereof, wherein:
X¹ is selected from N and CH;
R¹ is selected from hydroxyl, cyano, carboxyl, nitro, alkyl, haloalkyl, hydroxyalkyl, alkoxyl, cycloalkyloxy, heterocycloalkyloxy, cycloalkylalkoxy, heterocyclylalkoxy, cycloalkylalkyl, heterocyclylalkyl, monoalkylamino, dialkylamino, cycloalkylamino, heterocyclylamino, arylamino, heteroarylamino, cycloalkyl, heterocyclyl, aryl, heteroaryl, aryl fused to heterocyclyl, and heteroaryl fused to heterocyclyl, which are optionally substituted with one or more halogen, hydroxyl, amino, carboxyl, cyano, nitro, oxo, alkylsulfonyl, aminosulfonyl, alkylsulfonylalkyl, alkyl, cycloalkyl, heterocyclyl, alkylheterocyclyl, alkoxyl, haloalkyl, hydroxyalkyl, aminoalkyl, carboxyalkyl, cyanoalkyl, nitroalkyl, cycloalkylalkyl, heterocycloalkylalkyl, alkoxyalkyl, monoalkylamino, dialkylamino, alkylacyl, alkoxyacyl, alkylacyloxy, aminoacyl, alkenylacyl, monoalkylaminoalkenylacyl, dialkylaminoalkenylacyl, monoalkylaminoacyl, dialkylaminoacyl, alkylacylamino or alkylacylaminoalkyl;
R² and R³ are each independently selected from hydrogen, halogen, hydroxyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, alkenyl, alkynyl, cycloalkyl, heterocyclyl, aryl and heteroaryl, wherein the halogen, hydroxyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, alkenyl, alkynyl, cycloalkyl, heterocyclyl, aryl or heteroaryl are optionally substituted with one or more alkyl, haloalkyl, hydroxyl, hydroxyalkyl, halogen, oxo, alkoxyl, carboxyl, cyano, amino, monoalkylamino or dialkylamino;
R⁴ is selected from hydrogen, halogen, hydroxyl, alkyl, haloalkyl, hydroxyalkyl, alkoxyl, haloalkoxy, hydroxyalkoxy, nitro, carboxyl, cyano, amino, alkylamino, alkylacylamino, alkylacyl,aminoacyl, alkylaminoacyl, cycloalkyl, heterocycloalkyl, aryl, heteroaryl, cycloalkylamino, heterocycloalkylaryl, arylamino and heteroarylamino, wherein the halogen, hydroxyl, alkyl, haloalkyl, hydroxyalkyl, alkoxyl, haloalkoxy, hydroxyalkoxy, nitro, carboxyl, cyano, amino, alkylamino, alkylacylamino, alkylacyl, aminoacyl, alkylaminoacyl, cycloalkyl, heterocycloalkyl, aryl, heteroaryl, cycloalkylamino, heterocycloalkylaryl, arylamino and heteroarylamino are optionally substituted with one or more alkyl, alkoxyl, aryloxy, alkylamino, cycloalkyl, heterocycloalkyl, aryl, heteroaryl, arylamino, halogen, hydroxyl, amino, nitro, carboxyl, cyano, alkylacyl, aminoacyl, alkaminoacyl, sulfonyl or sulfhydryl;
R⁵ and R⁶ are each independently selected from hydrogen, halogen, hydroxyl,alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl;
m and n are each independently selected from1, 2 and 3.

In some particular embodiments, the compound of the present disclosure is a compound of general formula I or an isomer, pharmaceutically acceptable salt, solvate, crystal, isostere or prodrug thereof, wherein X¹ is N.

In other particular embodiments, the compound of the present disclosure is a compound of general formula I or an isomer,pharmaceutically acceptable salt, solvate, crystal, isostere or prodrug thereof, wherein X¹ is CH.

In some particular embodiments, the compound of the present disclosure is a compound of general formula I or an isomer, pharmaceutically acceptable salt, solvate, crystal, isostere or prodrug thereof, wherein R¹ is selected from hydroxyl, cyano, carboxyl, nitro, C₁₋₆alkyl, haloC₁₋₆alkyl, hydroxyC₁₋₆alkyl, C₁₋₆alkoxyl, C₃₋₆cycloalkyloxy, C₃₋₆heterocycloalkyloxy, C₃₋₆cycloalkylC₁₋₃alkoxyl, C₃₋₆heterocyclylC₁₋₃alkoxyl, C₃₋₆cycloalkylC₁₋₃alkyl, C₃₋₆heterocyclylC₁₋₃alkyl, monoC₁₋₆alkylamino, diC₁₋₆alkylamino, C₃₋₆cycloalkylamino, C₃₋₆heterocyclylamino, C₆₋₁₂arylamino, C₅₋₈heteroarylamino, C₃₋₆cycloalkyl, C₃₋₆heterocyclyl, C₆₋₁₂aryl, C₅₋₁₂heteroaryl, C₆₋₁₂aryl fused to C₃₋₁₀heterocyclyl and C₅₋₁₂heteroaryl fused to C₃₋₆heterocyclyl, which are optionally substituted with one or more halogen, hydroxyl, amino, carboxyl, cyano, nitro, oxo, C₁₋₆alkylsulfonyl, aminosulfonyl, C₁₋₆alkylsulfonylC₁₋₆alkyl, C₁₋₆alkyl, C₃₋₁₀cycloalkyl, C₃₋₁₀heterocyclyl, C₁₋₆alkylC₃₋₁₀heterocyclyl, C₁₋₆alkoxyl, haloC₁₋₆alkyl, hydroxyC₁₋₆alkyl, aminoC₁₋₆alkyl, carboxylC₁₋₆alkyl, cyanoC₁₋₆alkyl, nitroC₁₋₆alkyl, C₃₋₆cycloalkylC₁₋₆alkyl, C₃₋₆heterocycloalkylC₁₋₆alkyl, C₁₋₆alkoxyC₁₋₆alkyl, monoC₁₋₆alkylamino, diC₁₋₆alkylamino, C₁₋₆alkylacyl, C₁₋₆alkoxyacyl, C₁₋₆alkylacyloxy, aminoacyl, C₂₋₁₀alkenylacyl, monoC₁₋₆alkylaminoC₂₋₁₀alkenylacyl, diC₁₋₆alkylaminoC₂₋₁₀alkenylacyl, monoC₁₋₆alkylaminoacyl, diC₁₋₆alkylaminoacyl, C₁₋₆alkylacylamino or C₁₋₆alkylacylaminoC₁₋₆alkyl.

Preferably, R¹ is selected from hydroxyl, cyano, carboxyl, nitro, C₁₋₆alkyl, haloC₁₋₆alkyl, hydroxyC₁₋₆alkyl, C₁₋₆alkoxyl, C₃₋₆cycloalkyloxy, C₃₋₆heterocycloalkyloxy, C₃₋₆cycloalkylC₁₋₃alkoxyl, C₃₋₆heterocyclylC₁₋₃alkoxyl, C₃₋₆cycloalkylC₁₋₃alkyl, C₃₋₆heterocyclylC₁₋₃alkyl, monoC₁₋₆alkylamino, diC₁₋₆alkylamino, C₃₋₆cycloalkylamino, C₆₋₁₂arylamino, C₅₋₈heteroarylamino, C₃₋₆heterocyclylamino, C₃₋₆cycloalkyl, C₃₋₆heterocyclyl, C₆₋₁₂aryl, C₅₋₁₂heteroaryl, C₆₋₁₂aryl fused to C₃₋₁₀heterocyclyl, and C₅₋₁₂heteroaryl fused to C₃₋₆heterocyclyl, which are optionally substituted with one or more halogen, hydroxyl, amino, carboxyl, cyano, nitro, oxo, C₁₋₃alkylsulfonyl, aminosulfonyl, C₁₋₃alkylsulfonylC₁₋₃alkyl, C₁₋₃alkyl, C₃₋₆cycloalkyl, C₃₋₆heterocyclyl, C₁₋₃alkylC₃₋₆heterocyclyl, C₁₋₃alkoxyl, haloC₁₋₃alkyl, hydroxyC₁₋₃alkyl, aminoC₁₋₃alkyl, carboxylC₁₋₃alkyl, cyanoC₁₋₃alkyl, nitroC₁₋₃alkyl, C₃₋₆cycloalkylC₁₋₃alkyl, C₃₋₆heterocycloalkylC₁₋₃alkyl, C₁₋₃alkoxyC₁₋₃alkyl, monoC₁₋₃alkylamino, diC₁₋₃alkylamino, C₁₋₃alkylacyl,C₁₋₃alkoxyacyl, C₁₋₃alkylacyloxy, aminoacyl, C₂₋₆alkenylacyl, mono C₁₋₃alkylaminoC₂₋₆alkenylacyl, diC₁₋₃alkylaminoC₂₋₆alkenylacyl, monoC₁₋₃alkylaminoacyl, diC₁₋₃alkylaminoacyl, C₁₋₃alkylacylamino or C₁₋₃alkylacylaminoC₁₋₃alkyl.

Further preferably, R¹ is selected from hydroxyl, cyano, carboxyl, nitro, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy,I sopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, vinyl, propenyl, butenyl, 3-methyl-2-butenyl, ethynyl, propynyl, butynyl, pentynyl, cyclopropyl, cyclobutyl, cyclopentyl, cyclohexyl, 4- to 6-membered heterocycloalkyl, C₆₋₁₀aryl, 5- to 10-membered heteroaryl, C₆₋₁₂arylamino, C₅₋₈heteroarylamino, C₆₋₁₀aryl fused to 5- to 10-membered heterocyclyl, and 5- to 10-membered heteroaryl fused to 5- to 6-membered heterocyclyl, which are optionally substituted with one or more C₁₋₃alkyl, haloC₁₋₃alkyl, hydroxyl, hydroxyC₁₋₃alkyl, halogen, oxo, C₁₋₃alkoxyl, carboxyl, cyano, nitro, amino, C₁₋₃alkylsulfonyl, aminosulfonyl, C₁₋₃alkylsulfonylC₁₋₃alkyl, monoC₁₋₃alkylamino or diC₁₋₃alkylamino.

In some particular embodiments, the compound of the present disclosure is a compound of general formula I or an isomer, pharmaceutically acceptable salt, solvate, crystal, isostere or prodrug thereof, wherein:

R² and R³ are each independently selected from hydrogen, fluorine, chlorine, bromine, iodine, hydroxyl, C₁₋₆alkyl, haloC₁₋₆alkyl, hydroxyC₁₋₆alkyl C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino, C₂₋₁₀alkenyl, C₂₋₁₀alkynyl, C₃₋₁₀cycloalkyl, 3- to 10-membered heterocycloalkyl, C₆₋₁₈aryl and 5- to 18-membered heteroaryl, wherein the fluorine, chlorine, bromine, iodine, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino, C₂₋₁₀alkenyl, C₂₋₁₀alkynyl, C₃₋₁₀cycloalkyl, 3- to 10-membered heterocycloalkyl, C₆₋₁₈aryl and 5- to 18-membered heteroaryl are optionally substituted with one or more alkyl, haloalkyl, hydroxyl, hydroxyalkyl, halogen, oxo, alkoxyl, carboxyl, cyano, amino, monoalkylamino or dialkylamino. Preferably, R² and R³ are each independently selected from hydrogen, fluorine, chlorine, bromine, iodine, hydroxyl, methyl, ethyl, propyl, butyl, isopropyl, isobutyl, tertiary butyl, methylamino, ethylamino, propylamino, isopropylamino, cyclopropyl, cyclobutyl, cyclopentyl, cyclohexyl, phenyl, pyrrolyl, imidazolyl, pyrazolyl, thiazolyl, thienyl, furyl, pyridinyl, pyrazinyl, pyrimidyl, azetidinyl, oxetanyl, tetrahydropyrrolyl, tetrahydrofuranyl, piperidinyl, tetrahydropyranyl and morpholinyl, which are optionally substituted with one or more hydroxyl, methyl, ethyl, propyl, butyl, isopropyl, isobutyl, tertiary butyl, carboxyl, fluorine, chlorine, bromine, trifluoromethyl, trifluoroethyl, aminomethyl, aminoethyl, aminopropyl, methylamino, ethylamino, propylamino, isopropylamino, methoxy, ethoxy, propoxy, isopropoxy, oxo, formyl, acetyl, propanoyl, isopropionyl, vinyl, propenyl, ethynyl, propynyl, phenyl, naphthyl, pyrrolyl, imidazolyl, pyrazolyl, thiazolyl, thienyl, furyl, pyridinyl, pyrazinyl and pyrimidyl.

In some particular embodiments, the compound of the present disclosure is a compound of general formula I or an isomer, pharmaceutically acceptable salt, solvate, crystals, isosteres or prodrug thereof, wherein:

R⁴, R⁵ and R⁶ are each independently selected from hydrogen, halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino, and C₃₋₁₀cycloalkyl.

Preferably, R⁴ is selected from hydrogen, halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, C₁₋₆alkylamino, C₁₋₆alkylacylamino, C₁₋₆alkylacyl,aminoacyl, C₁₋₆alkylaminoacyl, C₃₋₈cycloalkyl, C₃₋₈heterocycloalkyl, C₆₋₁₂aryl, C₅₋₁₂heteroaryl, C₃₋₈cycloalkylamino, C₃₋₈heterocycloalkylaryl, C₆₋₁₂arylamino and C₅₋₈heteroarylamino, wherein the halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, C₁₋₆alkylamino, C₁₋₆alkylacylamino, C₁₋₆alkylacyl, aminoacyl, C₁₋₆alkylaminoacyl, C₃₋₈cycloalkyl, C₃₋₈heterocycloalkyl, C₆₋₁₂aryl, C₅₋₁₂heteroaryl, C₃₋₈cycloalkylamino, C₃₋₈heterocycloalkylaryl, C₆₋₁₂arylamino and C₅₋₈heteroarylamino are optionally substituted with one or more C₁₋₆alkyl, C₁₋₆alkoxyl, C₆₋₁₂aryloxy, C₁₋₆alkylamino,C₃₋₆cycloalkyl, C₃₋₆heterocycloalkyl, C₆₋₁₂aryl, C₅₋₈heteroaryl, C₆₋₁₂arylamino, halogen, hydroxyl, amino, nitro, carboxyl, cyano, alkylacyl, aminoacyl, alkaminoacyl, sulfonyl or sulfhydryl; R⁵ and R⁶ are each independently selected from hydrogen, fluorine, chlorine, bromine, iodine, hydroxyl, methyl, ethyl, propyl, isopropyl, trifluoromethyl, rifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl, and cyclohexyl.

In some preferred embodiments, the compound of general formula I of the present disclosure is a compound of the following general formula Ia or an isomer, pharmaceutically acceptable salt, solvate, crystal, isostere or prodrug thereof, 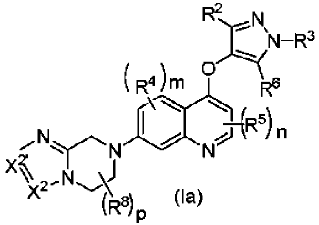 wherein R², R³, R⁴, R⁵, R⁶, m, and n are defined as in claims 1-5;
X² and X^{2'} are each independently selected from N and C(R⁷), wherein R⁷ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl;
R⁸ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl; and
p is selected from 1, 2 and 3.

In some particular embodiments, provided is the compound of general formula Ia or the isomer, pharmaceutically acceptable salt, solvate, crystal, isostere or prodrug thereof according to the present disclosure, in which X² and X^{2'} are each independently selected from N and C(R⁷); preferably, X² is N and X^{2'} is C(R⁷) or X² is C(R⁷) and X^{2'} is N, wherein R⁷ is selected from hydrogen, halogen, hydroxyl, oxo, C₁₋₆alkylsulfonyl, C₁₋₆alkylsulfonylC₁₋₆alkyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino and C₃₋₆cycloalkyl; R⁸ is selected from hydrogen, halogen, hydroxyl, oxo, C₁₋₆alkylsulfonyl, C₁₋₆alkylsulfonylC₁₋₆alkyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino and C₃₋₆cycloalkyl; and p is selected from 1, 2 and 3.

In some preferred embodiments, the compound of general formula I of the present disclosure is a compound of the fowllowing general formula Ib or an isomer, pharmaceutically acceptable salt, solvate, crystal, isostere or prodrug thereof, 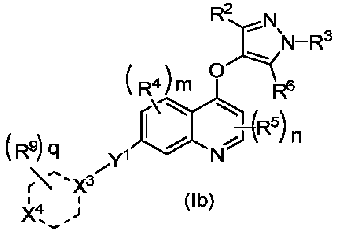 wherein R², R³, R⁴, R⁵, R⁶, X¹, m, and n are defined as in general formula I;
X³ and X⁴ are each independently selected from N, C, CH, CH₂, O and S;
Y¹ is selected from NH, C(O)NH or absent;
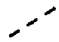 is a single bond or a double bond;
R⁹ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, cycloalkyl; Preferably, R⁹ is selected from hydrogen, fluorine, chlorine, bromine, iodine, hydroxyl, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl and cyclohexyl; and
q is selected from 1, 2, 3, 4 and 5.

In some preferred embodiments, the compound of general formula I of the present disclosure is a compound of the fowllowing general formula Ic or an isomer, pharmaceutically acceptable salt, solvate, crystal, isostere or prodrug thereof, 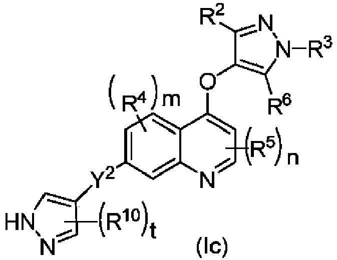 wherein R², R³, R⁴, R⁵, R⁶, X¹, m, and n are defined as in general formula I;
Y² is selected from NH, C(O)NH or absent;
R¹⁰ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, cycloalkyl; preferably, R¹⁰ is selected from hydrogen, fluorine, chlorine, bromine, iodine, hydroxyl, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl, and cyclohexyl; and
t is selected from 1, 2 and 3.

In some preferred embodiments, the compound of the present disclosure is an compound of general formula I, Ia, Ib or Ic or an isomer, pharmaceutically acceptable salt, solvate, crystal, isostere or prodrug thereof, wherein:
R¹ is selected from cyclopentyl, cyclohexyl, 4- to 6-membered heterocycloalkyl, C₆₋₁₀aryl, 5- to 10-membered heteroaryl, C₆₋₁₂arylamino, C₅₋₈heteroarylamino, C₆₋₁₀aryl fused to 5- to 10-membered heterocyclyl, and 5- to 10-membered heteroaryl fused to 5- to 6-membered heterocyclyl, which are optionally substituted with one or more C₁₋₃alkyl, haloC₁₋₃alkyl, hydroxyl, hydroxyC₁₋₃alkyl, halogen, oxo, C₁₋₃alkoxyl, carboxyl, cyano, amino, monoC₁₋₃alkylamino or diC₁₋₃alkylamino;
R² and R³ are each independently selected from hydrogen, fluorine, chlorine, bromine, methyl, ethyl, propyl,butyl, isopropyl, isobutyl, tertiary butyl, methylamino, ethylamino, propylamino, isopropylamino, cyclopropyl, cyclobutyl, cyclopentyl, cyclohexyl,phenyl, pyrrolyl, imidazolyl, pyrazolyl, thiazolyl, thienyl, furyl, pyridinyl, pyrazinyl, pyrimidyl, azetidinyl, oxetanyl, tetrahydropyrrolyl, tetrahydrofuranyl, piperidinyl, tetrahydropyranyl and morpholinyl, which are optionally substituted with one or more hydroxyl, methyl, ethyl, propyl, butyl, isopropyl, isobutyl, tertiary butyl, carboxyl, fluorine, chlorine, bromine, trifluoromethyl, trifluoroethyl, aminomethyl, aminoethyl, aminopropyl, methylamino, ethylamino, propylamino, isopropylamino, methoxy, ethoxy, propoxy, isopropoxy, oxo, formyl, acetyl, propanoyl, isopropionyl, vinyl, propenyl, ethynyl, or propynyl;
R⁴ is selected from hydrogen, hydrogen, fluorine, chlorine, bromine, hydroxyl, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl, cyclohexyl, 4- to 6-membered heterocycloalkyl, C₆₋₁₀aryl, 5- to 10-membered heteroaryl, C₆₋₁₂arylamino, C₅₋₈heteroarylamino, C₆₋₁₀aryl, C₃₋₈cycloalkylamino, C₃₋₈heterocycloalkylaryl, C₆₋₁₂arylamino, C₅₋₈heteroarylamino, formamyl, aminoacetyl, which are optionally substituted with one or more C₁₋₆alkyl, C₁₋₆alkoxyl, C₆₋₁₂aryloxy, C₁₋₆alkylamino,C₃₋₆cycloalkyl, C₃₋₆heterocycloalkyl, C₆₋₁₂aryl, C₅₋₈heteroaryl, C₆₋₁₂arylamino, halogen, hydroxyl, amino, nitro, carboxyl, cyano, alkylacyl, aminoacyl, alkaminoacyl, sulfonyl or sulfhydryl;
R⁵ and R⁶ are each independently selected from hydrogen, fluorine, chlorine, bromine, hydroxyl, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl, and cyclohexyl;
R⁸, R⁹, R¹⁰ are each independently selected from hydrogen, fluorine, chlorine, bromine, iodine, hydroxyl, methyl, ethyl, propyl, isopropyl, trifluoromethyl, trifluoroethyl, hydroxymethyl, hydroxyethyl, hydroxypropyl, 2-hydroxypropyl, methoxy, ethoxy, propoxy, isopropoxy, nitro, carboxyl, cyano, amino, methylamino, ethylamino, propylamino, isopropylamino, dimethylamino, diethylamino, methylethylamino, dipropylamino, methylpropylamino, ethylpropylamino, methylacylamino, ethylacylamino, vinylacylamino, methylacyl, ethylacyl, vinylacyl, aminoacyl, methylaminoacyl, ethylaminoacyl, cyclopropyl, cyclobutyl, cyclopentyl, and cyclohexyl;
X¹ is N;
X² is N and X^{2'} is C(R⁷), or X² is C(R⁷) and X^{2'} is N, wherein R⁷ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl;
X³ and X⁴ are each independently selected from N, C, CH, CH₂, O and S;
Y¹ and Y² are each independently selected from NH and absent; and
m, n, p, and q are each independently selected from 1 and 2.

In some embodiments, in the compound or the pharmaceutically acceptable salt, isomer, solvate, crystal or prodrug thereof accoding to the present disclosure, general formula I has a structure of the following general formula Id, 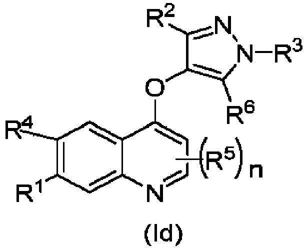 wherein R¹, R², R³, R⁴, R⁵, R⁶ and n are defined as in general formula I.

In some particular embodiments, the compound of the present disclosure is a compound of general formula I or Id or an isomer, pharmaceutically acceptable salt, solvate, crystal, isostere or prodrug thereof, wherein R¹ is selected from C₁₋₆alkoxyl, morpholinyl, piperidinyl, pyrazolyl, phenyl, pyridinyl, pyridineamino, pyrrolopyrazolyl, triazolopyrazinyl, thiomorpholinyl, which are optionally substituted with one or more halogen, hydroxyl, amino, carboxyl, cyano, nitro, oxo, C₁₋₆alkylsulfonyl, aminosulfonyl, C₁₋₆alkylsulfonylC₁₋₆alkyl, C₁₋₆alkyl, C₃₋₁₀cycloalkyl, C₃₋₁₀heterocyclyl, C₁₋₆alkylC₃₋₁₀heterocyclyl, C₁₋₆alkoxyl, haloC₁₋₆alkyl, hydroxyC₁₋₆alkyl, aminoC₁₋₆alkyl, carboxylC₁₋₆alkyl, cyanoC₁₋₆alkyl, nitroC₁₋₆alkyl, C₃₋₆cycloalkylC₁₋₆alkyl, C₃₋₆heterocycloalkylC₁₋₆alkyl, C₁₋₆alkoxyC₁₋₆alkyl, monoC₁₋₆alkylamino, diC₁₋₆alkylamino, C₁₋₆alkylacyl, C₁₋₆alkoxyacyl, C₁₋₆alkylacyloxy, aminoacyl, C₂₋₁₀alkenylacyl, monoC₁₋₆alkylaminoC₂₋₁₀alkenylacyl, diC₁₋₆alkylaminoC₂₋₁₀alkenylacyl, monoC₁₋₆alkylaminoacyl, diC₁₋₆alkylaminoacyl, C₁₋₆alkylacylamino or C₁₋₆alkylacylaminoC₁₋₆alkyl;
R² and R³ are each independently selected from hydrogen, fluorine, chlorine, bromine, iodine, hydroxyl, methyl, ethyl, propyl, butyl, isopropyl, isobutyl, tertiary butyl, methylamino, ethylamino, propylamino, isopropylamino, cyclopropyl, cyclobutyl, cyclopentyl, cyclohexyl, phenyl, pyrrolyl, imidazolyl, pyrazolyl, thiazolyl, thienyl, furyl, pyridinyl, pyrazinyl, pyrimidyl, azetidinyl, oxetanyl, tetrahydropyrrolyl, tetrahydrofuranyl, piperidinyl, tetrahydropyranyl and morpholinyl, which are optionally substituted with one or more hydroxyl, methyl, ethyl, propyl, butyl, isopropyl, isobutyl, tertiary butyl, carboxyl, fluorine, chlorine, bromine, trifluoromethyl, trifluoroethyl, aminomethyl, aminoethyl, aminopropyl, methylamino, ethylamino, propylamino, isopropylamino, methoxy, ethoxy, propoxy, isopropoxy, oxo group, formyl, acetyl, propanoyl, isopropionyl, vinyl, propenyl, ethynyl, propynyl, phenyl, naphthyl, pyrrolyl, imidazolyl, pyrazolyl, thiazolyl, thienyl, furyl, pyridinyl, pyrazinyl and pyrimidyl; and
R⁴ is selected from hydrogen, aminoacyl and pyrazolamino, wherein the aminoacyl and pyrazolamino are optionally substituted with one or more C₁₋₆alkyl, C₁₋₆alkoxyl, C₆₋₁₂aryloxy, C₁₋₆alkylamino, C₃₋₆cycloalkyl, C₃₋₆heterocycloalkyl, C₆₋₁₂aryl, C₅₋₈heteroaryl, C₆₋₁₂arylamino, halogen, hydroxyl, amino, nitro, carboxyl, cyano, alkylacyl, aminoacyl, alkaminoacyl, sulfonyl or sulfhydryl.

In one embodiment, in the compound of general formula I or Id or the isomer, pharmaceutically acceptable salt, solvate, crystal, isostere or prodrug thereof according to the present disclosure, R⁴ is substituted with oxetanyl.

In some embodiments, the compound of the present disclosure is an compound of general formula I or Id or an isomer, pharmaceutically acceptable salt, solvate, crystal, isostere or prodrug thereof, wherein X¹ is N, R¹ is selected from the following groups: 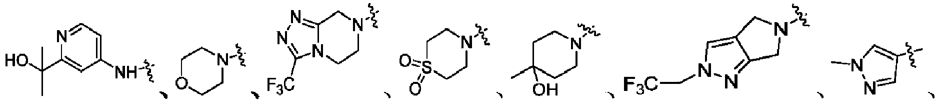 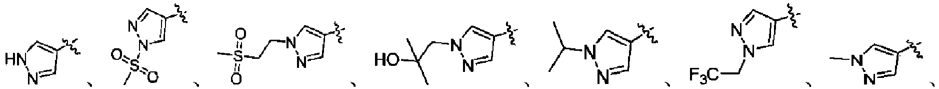 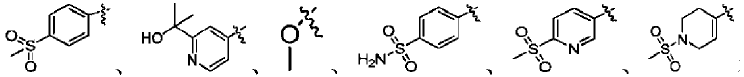 and 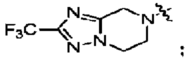 R² is tetrahydropyranyl; R³ is selected from C₁₋₆alkyl and C₃₋₆cycloalkyl; R⁴ is selected from H,

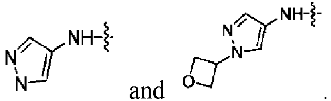

The present disclosure provides the following specific compounds or pharmaceutically acceptable salts, isomers, solvates, crystals or prodrugs thereof:

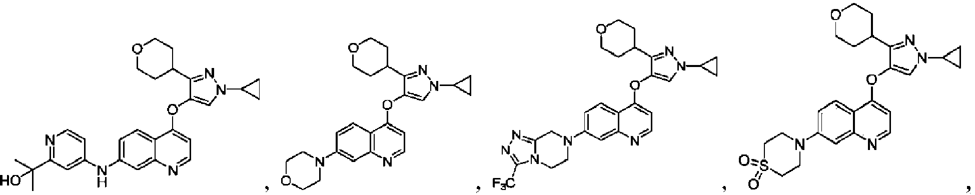

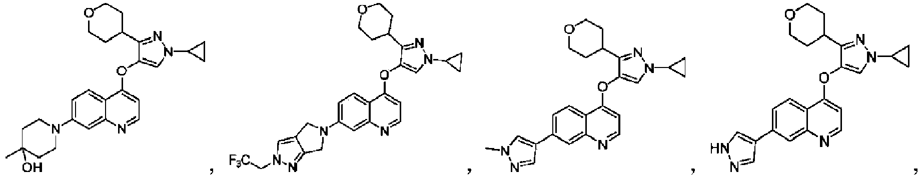

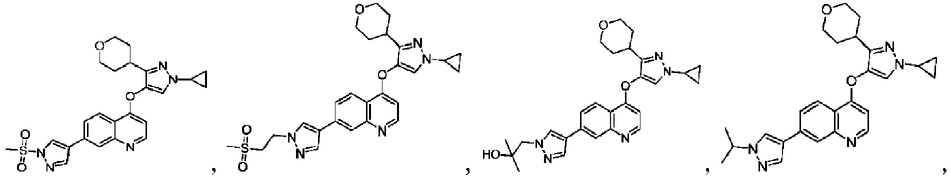

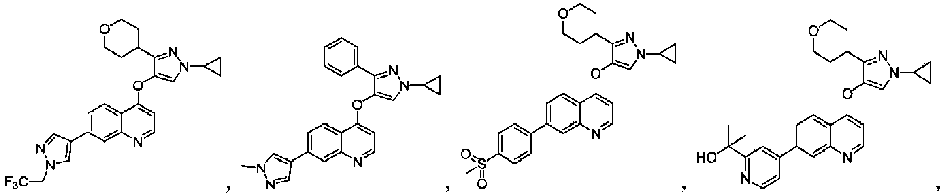

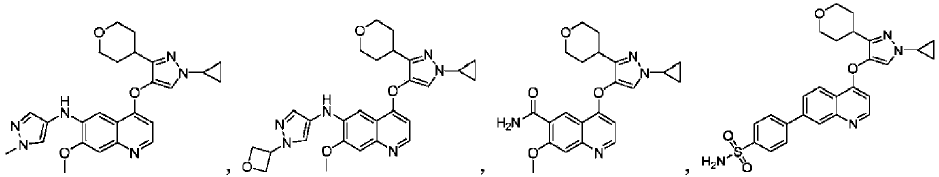

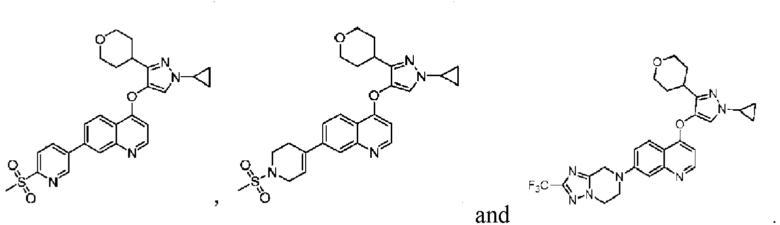

In another aspect, the present disclosure provides a preparation method of the compounds of the general formulas or the pharmaceutically acceptable salts, isomers, solvates, crystals or prodrugs thereof of the present disclosure, which includes: 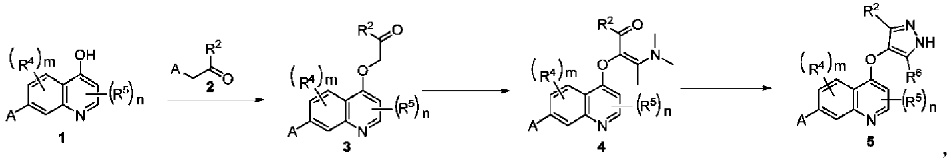 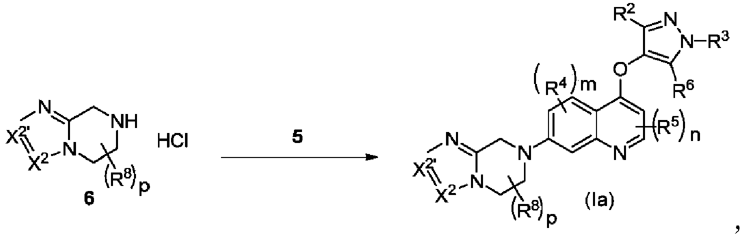 or 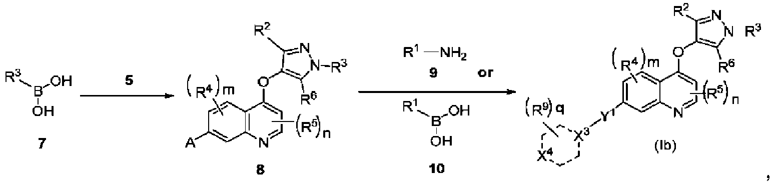 or 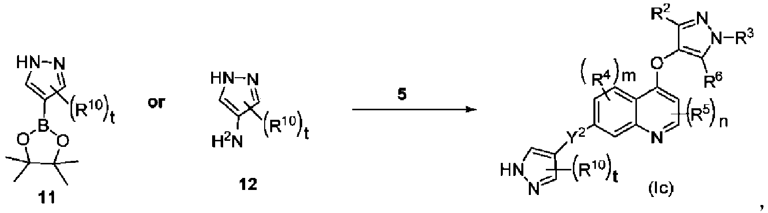
1) reacting a compound of Fomula 1 with a compound of Fomula 2 to obtain a compound of Fomula 3;
2) performing a reaction of the compound of Fomula 3 to obtain a compound of Fomula 4;
3) performing a reaction of the compound of Fomula 4 to obtain a compound of Fomula 5;
4) reacting the compound of Fomula 5 with a compound of Fomula 6 to obtain a compound of Fomula Ia; or
5) reacting the compound of Fomula 5 with a compound of Fomula 7 to obtain a compound of Fomula 8;
6) reacting the compound of Fomula 8 with a compound of Fomula 9 or 10 to obtain a compound of Fomula Ib; or
7) reacting the compound of Fomula 5 with a compound of Fomula 11 or 12 to obtain a compound of Fomula Ic.
wherein R¹, R², R³, R⁴, R⁵, R⁶, R⁸, R⁹, R¹⁰, X¹, X², X^{2'}, X³, X⁴, Y¹, Y², 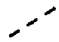 , m, n, p, q and t have definitions as in general formula I; A is halogen, and preferably bromine.

In the third aspect, the present disclosure provides a pharmaceutical composition, which comprises the compound or the isomer, pharmaceutically acceptable salt, solvate, crystal, isostere or prodrug thereof of the present disclosure.

In some embodiments, the pharmaceutical composition provided by the present disclosure comprises the compound or the isomer, pharmaceutically acceptable salt, solvate, crystal, isostere or prodrug thereof of the present disclosure, and further comprises one or more agents selected from the group consisting of a TGF-βR1 inhibitor, a tyrosine proteinase inhibitor, an EGFR inhibitor, a VEGFR inhibitor, a Bcr-Abl inhibitor, a c-kit inhibitor, a c-Met inhibitor, an Raf inhibitor, an MEK inhibitor, a histone deacetylase inhibitor, an IDH inhibitor, a VEGF antibody, an EGF antibody, an HIV protein kinase inhibitor, an HMG-CoA reductase inhibitor and the like.

In some embodiments, the present disclosure provides the use of the compound or the isomer, pharmaceutically acceptable salt, solvate, crystal, isostere or prodrug thereof of the present disclosure, and the pharmaceutical composition comprising the compound or the isomer, pharmaceutically acceptable salt, solvate, crystal, isostere or prodrug thereof of the present disclosure, and the compound or the pharmaceutical composition, in a medicament for treating and/or preventing diseases related to TGF-βR1.

The compound or the isomer, pharmaceutically acceptable salt, solvate, crystal, isostere or prodrug thereof of the present disclosure may be mixed with a pharmaceutically acceptable carrier, a diluent or an excipient to prepare a pharmaceutical preparation, so as to be suitable for oral or parenteral administration. Administering methods include, but are not limited to intradermal, intramuscular, intraperitoneal, intravenous, subcutaneous, intranasal and oral routes. The preparation can be administered by any routes, for example, by infusion or push injection, or by absorption across the epithelia or skin mucosa (for example, oral mucosa or rectum). The administration may be systemic or topical. Examples of preparations for oral administration include solid or liquid dosage forms, and specifically include tablets, pills, granules, powders, capsules, syrups, emulsions, suspensions, and the like. The preparation may be made using a method known in the art, and contains a carrier, diluent or excipient conventionally used in the field of pharmaceutical preparations.

In the forth aspect, the present disclosure provides the use of the compound represented by general formula I, Ia, Ib, Ic or Id or the isomer, pharmaceutically acceptable salt, solvate, crystal, isostere or prodrug thereof of the present disclosure, or the pharmaceutical composition comprising the same, in the manufacture of a medicament for treating and/or preventing cancer, tissue hyperplasia diseases, fibrotic or inflammatory diseases, in which the conditions of cancer, tissue hyperplasia diseases and fibrotic or inflammatory diseases include, but are not limited to melanoma, papillary thyroid neoplasm, bile duct cancer, colon cancer, ovarian cancer, lung cancer, malignant lymphoma; carcinomas and sarcomas of liver, kidney, bladder, prostate, breast and pancreas; as well as primary and recurrent solid tumors of skin, colon, thyroid, lung and ovary, or leukocythemia, glioblastoma (neuroglioma), myelodysplastic syndrome (MDS), myeloproliferative neoplasm (MPN), acute myeloid leukemia (AML), sarcomas, non-small cell lung cancer, chondrosarcoma, bile duct cancer or angioimmunoblastic lymphadenopathy, liver fibrosis and chronic kidney diseases.

### Term definations

Unless stated to the contrary, the terms used in the specification and CLAIMS have the following meanings.

The "hydrogen", "carbon" and "oxygen" in the compounds of the present disclosure include all their isotopes. Isotopes should be understood to include those atoms that have the same atomic number but different mass numbers. For example, isotopes of hydrogen include tritium and deuterium, isotopes of carbon include ¹³C and ¹⁴C, and isotopes of oxygen include ¹⁶O and ¹⁸O.

The "halogen" in the present disclosure refers to fluorine, chlorine, bromine, iodine. The "halo" in the present disclosure refers to substitution by fluorine, chlorine, bromine or iodine.

The "alkyl" in the present disclosure refers to a straight- or branched-chain saturated aliphatic hydrocarbon group, preferably a straight- or branched-chain group containing 1 to 6 carbon atoms, more preferably a straight- or branched-chain group containing 1 to 3 carbon atoms. Their non-limiting examples include methyl, ethyl, n-propyl, isopropyl and the like. The alkyl may be substituted or unsubstituted, and when substituted, the substituent may be at any available point of attachment.

The "haloalkyl" in the present disclosure refers to an alkyl group substituted with at least one halogen.

The "hydroxyalkyl" in the present disclosure refers to an alkyl group substituted with at least one hydroxyl.

The "alkoxy" in the present disclosure refers to an -O-alkyl group. Non-limiting examples of alkoxy groups include: methoxy, ethoxy, propoxy, n-propoxy, isopropoxy and the like. The alkoxy group may be optionally substituted or unsubstituted, and when substituted, the substituent may be at any available point of attachment.

The "cycloalkyl" in the present disclosure refers to a cyclic saturated hydrocarbon group, such as cyclopropyl and cyclobutyl.

The "heterocyclyl" in the present disclosure refers to a radical derived from a 3- to 12-membered non-aromatic ring system ("3-12 membered heterocyclyl") containing 1 to 4 heteroatoms in the ring (wherein each heteroatom is independently selected from nitrogen, oxygen, sulfur, boron, phosphorus and silicon). In the heterocyclyl groups containing one or more nitrogen atoms, the point of attachment may be a carbon or nitrogen atom, as long as the valence allows. The heterocyclyl groups may either be monocyclic ("monocyclic heterocyclyl") or fused, bridged or spiro ring systems (e.g., bicyclic systems ("bicyclic heterocyclyl")), and may be saturated or partially unsaturated. Suitable saturated and partially saturated heterocyclyl redicals include, but are not limited to, tetrahydrofuranyl, tetrahydrothienyl, pyrrolidinyl, piperidinyl, piperazinyl, morpholinyl, isoxazolinyl and the like. The heterocyclyl bicyclic ring system may contain one or more heteroatoms in one or two rings. "Heterocyclyl" also includes ring systems wherein the heterocyclic ring, as defined above, is fused to one or more carbocyclyl groups (wherein the point of attachment may be at the carbocyclyl or at the heterocyclic ring); or the heterocyclic ring in the ring system, as defined above, is fused to one or more aryl or heteroaryl (wherein the point of attachment is at the heterocyclic ring). In addition, in such cases, the number of ring members continues to be referred to as the number of ring members in the ring system. Unless otherwise specified, each instance of heterocyclyl is independently optionally substituted, that is, unsubstituted ("unsubstituted heterocyclyl") or substituted with one or more substituents ("substituted heterocyclyl"), such as substituted or unsubstituted piperidinyl, substituted or unsubstituted bridged ring morpholinyl, and the like. In certain instances, the heterocyclyl group is a substituted 4- to 10- membered heterocyclyl. Exemplary 5-membered heterocyclyl groups fused to a C₆ aryl ring include, but are not limited to, indolinyl, isoindolinyl, dihydrobenzofuranyl, dihydrobenzothienyl, benzoxazolonyl and the like. Exemplary 6-membered heterocyclyl groups fused to a C₆ aryl ring include, but are not limited to, tetrahydroquinolinyl, tetrahydroisoquinolinyl, and the like. Exemplary 5- and 6-membered heterocycloalkyl groups fused to a 5-membered heteroaromatic ring include, but are not limited to 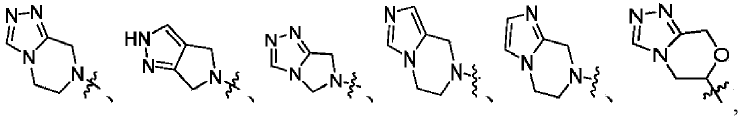 and the like.

The "aryl" in the present disclosure refers to an aromatic system that may contain a single ring or a fused polycyclic ring, preferably an aromatic system that contains a single ring or a fused bicyclic ring, and the aryl contains 6 to 18 carbon atoms, preferably about 6 to about 12 carbon atoms. Suitable aryl groups include, but are not limited to, phenyl, naphthyl, anthryl, tetrahydronaphthyl, fluorenyl, and indanyl. The aryl groups may be optionally substituted or unsubstituted, and when substituted, the substituent may be at any available point of attachment.

The "heteroaryl" (by itself or being in any combinatorial groups, such as "heteroarylamino") in the present disclosure refers to an aryl group wherein at least one carbon atom is replaced by a heteroatom which may be O, S and N, and consists of 5-20 atoms (5- to 20-membered heteroaryl), further preferably consists of 5-12 atoms (5- to 12-membered heteroaryl), and includes but are not limited to imidazolyl, benzimidazolyl, imidazopyridinyl, quinazolinol, pyrrolyl, imidazolone group, furyl, thienyl, pyrazolyl, oxazolyl, thiazolyl, isoxazolyl, isothiazolyl, oxadiazolyl, triazolyl, pyrimidyl, pyridinyl, pyrazinyl, pyridazinyl, pyrimidopyrazolyl, pyrimidoimidazolyl and the like. Heteroaryl groups can be optionally substituted or unsubstituted, and when substituted, the substituents can be at any available point of attachment.

The "isomers" in the present disclosure are compounds with identical molecular formulas but differing in properties or bonding sequences of their atoms or spatial arrangements of their atoms. Stereoisomers are isomers that differ in spatial arrangements of their atoms. Stereoisomers that are not mirror images of each other are diastereomers, and stereoisomers that are non-superimposable mirror images of each other are enantiomers. When a compound has an asymmetric center, for example, when it is bonded to four different groups, a pair of enantiomers is possible. Enantiomers are characterized by the absolute configuration of their asymmetric centers and are described and designated as dextrorotatory or levorotatory (that is, as (+) or (-)-isomer, respectively) by the Cahn-Prelog R- and S-sequence rules, or by the method of molecule rotating the plane of polarized light. Chiral compounds may be present as single enantiomers or mixtures thereof. A mixture containing the enantiomers in equal proportions is called a "racemic mixture".

The "pharmaceutically acceptable salt" of the present disclosure refers to a salt of the compound of the present disclosure. Such salts are safe and effective when used in the mammal body, and has due biological activity.

The "solvate" in the present disclosure refers to a complex formed by a combination of a solute (such as an active compound, or a salt of an active compound) and a solvent (such as water) in a conventional sense. The solvent refers to a solvent known or easily determined by those skilled in the art. If it is water, the solvate is usually referred to as a hydrate, such as hemihydrate, monohydrate, dihydrate, trihydrate, or alternative amounts thereof.

The bioisostere (or simply "isostere") in the present disclosure, a term generally recognized in the art, is used to define drug analogs in which one or more atoms (or atomic groups) have been replaced by the substitutional atoms (or atomic groups) with similar spatial and/or electronic characteristics to these atoms replaced.

The "crystal" in the present disclosure refers to a solid whose internal structure is formed by regularly repeating constituent atoms (or groups thereof) in three dimensions, which is different from an amorphous solid that does not have such a regular internal structure.

The "prodrug" refers to a compound that transfers to the compound of Formula I, Ia, Ib, Ic, or Id by reacting with enzymes, gastric acid, etc., under physiological conditions in an living organism.

The "pharmaceutical composition" in the present disclosure refers to a mixture containing any one of the compounds described herein, including corresponding isomers, prodrugs, solvates, pharmaceutically acceptable salts or chemically protected forms thereof, and one or more pharmaceutically acceptable carriers.

The "excipient" in the present disclosure refers to an inert substance that is added to a pharmaceutical composition to further facilitate the administration of a compound. Excipients may include calcium carbonate, calcium phosphate, various sugars and various types of starch, cellulose derivatives, gelatin, plant oil, and polyethylene glycol.

The "pharmaceutically acceptable carrier" in the present disclosure refers to a carrier that does not cause significant irritation to organisms and does not interfere with the biological activity and properties of the administered compound, including all solvents, diluents or other excipients, dispersants, surfactants, isotonic agents, thickeners or emulsifiers, preservatives, solid binders, lubricants, etc. Unless any conventional carrier medium is incompatible with the compounds of the present disclosure, some examples of pharmaceutically acceptable carriers may include, but are not limited to, sugars, such as lactose; starch, such as corn starch; cellulose and its derivatives, such as sodium carboxymethyl cellulose, malt, and gelatin.

DMSO involved in "¹HNMR (400 MHz, DMSO)" of the present disclosure refers to hexadeuterated dimethyl sulfoxide, e.g., DMSO-d₆.

### DETAILED DESCRIPTION

The following representative examples are given to better illustrate the present disclosure, rather than to limit the protection scope of the present disclosure. The materials used in the following examples are all commercially available unless otherwise specified.

### Example 1 2-(4-((4-((1-cyclopropyl-3-(tetrahydro-2H-pyran-4-yl)-1H-pyrazol-4-yl)oxy)quinolin-7-yl)a mino)pyridin-2-yl)propan-2-ol

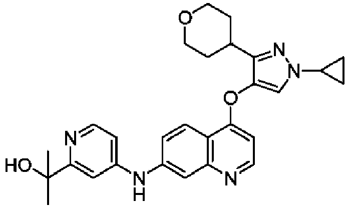

### Step 1: Synthesis of 2-((7-bromoquinolin-4-yl)oxy)-1-(tetrahydro-2H-pyran-4-yl)ethan-1-one

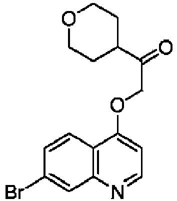

7-bromo-4-hydroxyquinoline (2 g, 8.9 mmol) and acetone (20 mL) were placed in a reaction flask, and to the above reaction flask 2-bromo-1-(tetrahydro-2*H*-pyran-4-yl)ethanone (2.3 g, 11.1 mmol) and potassium carbonate (1.53 g, 11.1 mmol) were added, to react for 5 h while the reaction system was stirred at 40°C. After the reaction was completed, it was filtered. The filtrate was concentrated under reduced pressure, and purified by column chromatography to obtain 665 mg of the title compound. LC-MS m/z: 350.1, 352.1[M+H]⁺.

### Step 2: Synthesis of (E)-2-((7-bromoquinolin-4-yl)oxy)-3-(dimethylamino)-1-(tetrahydro-2H-pyran-4-yl)prop-2-en-1-one

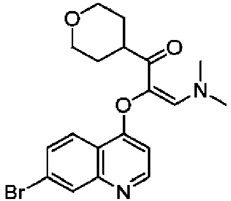

2-((7-bromoquinolin-4-yl)oxy)-1-(tetrahydro-2*H*-pyran-4-yl)ethan-1-one (665 mg, 1.9 mmol) and *N*,*N-*dimethylformamide dimethyl acetal (2 mL) were placed in a reaction flask, to react at 100°C for 2 h while the system was stirred. After the reaction was completed, the resulting product was cooled down to room temperature, and concentrated under reduced pressure. The residue was dissolved in ethyl acetate (40 mL), and washed with water (50 mL) and a saturated aqueous solution of sodium chloride (50 mL). The organic phase was dried with anhydrous sodium sulfate, and concentrated under reduced pressure to obtain the title compound which was directly introduced to the next step.

### Step 3: Synthesis of 7-bromo-4-((3-(tetrahydro-2H-pyran-4-yl)-1H-pyrazol-4-yl)oxy)quinoline

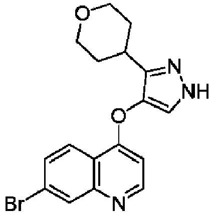

(*E*)-2-((7-bromoquinolin-4-yl)oxy)-3-(dimethylamino)-1-(tetrahydro-2*H*-pyran-4-y l)prop-2-en-1-one (665 mg, 1.64 mmol) and acetic acid (10 mL) were placed in a reaction flask, and cooled down to 0°C. Hydrazine-hydrate (0.5mL) was added, and allowed to return to room temperature, to react overnight under nitrogen atmosphere with stirring. The mixture was poured into an ice water mixture (25 mL), and extracted with ethyl acetate. The organic phase was combined, and washed with water (20 mL), saturated sodium bicarbonate and saturated brine (100 mL) successively, dried with anhydrous sodium sulfate, concentrated under reduced pressure, and purified by column chromatography to obtain 350 mg. LC-MS m/z: 374.0, 376.0 [M+H]⁺.

### Step 4: Synthesis of 7-bromo-4-((1-cyclopropyl-3-(tetrahydro-2H-pyran-4-yl)-1H-pyrazol-4-yl)oxy)quinoline

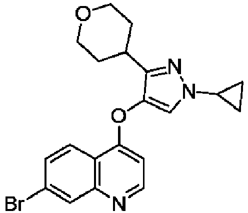

In a reaction flask, 2,2-bipyridine (46 mg, 0.3 mmol) and copper acetate (54 mg, 0.3 mmol) were placed, 1,2-dichloroethane (2 mL) was added, and refluxed at 75°C for 25 minutes, and then cooled down to room temperature. A 1,2-dichloroethane (1mL) solution of 7-bromo-4-((3-(tetrahydro-2*H*-pyran-4-yl)-1*H*-pyrazol-4-yl)oxy)quinoline (100 mg, 0.26 mmol) was added, and cyclopropylboronic acid (46 mg, 0.52 mmol) and sodium carbonate (57 mg, 0.52 mmol) were added at last. The reaction system was stirred to react for 2 h at 75°C under an oxygen atmosphere, and cooled down to room temperature. The reaction solution was diluted with ethyl acetate (20 mL), filtered through a short column packed with silica gel, and rinsed with ethyl acetate (50 mL). The filtrate was washed with water and saturated brine, dried with anhydrous sodium sulfate, and concentrated under reduced pressure to obtain 88 mg of the title compound. LC-MS m/z: 414.1, 416.1[M+H]⁺.

### Step 5: Synthesis of 2-(4-((4-((1-cyclopropyl-3-(tetrahydro-2H-pyran-4-yl)-1H-pyrazol-4-yl)oxy)quinolin-7-yl)a mino)pyridin-2-yl)propan-2-ol

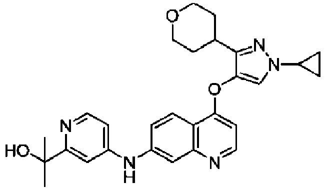

In a reaction flask, 7-bromo-4-((1-cyclopropyl-3-(tetrahydro-2*H*-pyran-4-yl)-1*H*-pyrazol-4-yl)oxy)quinoline (88 mg, 0.21 mmol), 2-(4-amino-2-pyridinyl)propane-2-ol hydrochloride (48 mg, 0.25 mmol), sodium tert-butoxide (60 mg, 0.63 mmol), 2-(dicyclohexylphosphino)-2',4',6'-triisopropylbiphenyl (8 mg, 0.017 mmol) and tris(dibenzylideneacetone)dipalladium(0) (4 mg, 0.004 mmol) were placed, and dissolved by adding 2 mL of tert-butyl alcohol, reacted overnight Under an argon atmosphere at 100°C with stirring, and cooled down to room temperature. 50 mL ethyl acetate and 10 mL of water were added to extract. The organic phase was dried with anhydrous sodium sulfate, concentrated under reduced pressure, and purified by column chromatography to obtain 12 mg of the title compound. LC-MS m/z: 486.2[M+H]⁺. ¹HNMR(400 MHz, DMSO) δ 10.06(s, 1H), 8.66(d, *J*=5.1 Hz, 1H), 8.29(d, *J*=8.9 Hz, 1H), 8.24(d, *J*=6.1 Hz, 1H), 7.96(s, 1H), 7.82(s, 1H), 7.57(d, *J*=8.8 Hz, 1H), 7.44(s, 1H), 7.11(s, 1H), 6.68(d, *J*=5.0 Hz, 1H), 5.59(s, 1H), 3.79(d, *J*=11.1 Hz, 2H), 3.76-3.60(m, 1H), 3.29-3.17(m, 2H), 2.76(dt, *J*=15.5, 7.9 Hz, 1H), 1.81-1.56(m, 4H), 1.48(s, 6H), 1.06(d, *J*=3.1 Hz, 2H), 0.96(d, *J*=5.3 Hz, 2H).

### Example 2 4-(4-((1-cyclopropyl-3-(tetrahydro-2H-pyran-4-yl)-1H-pyrazol-4-yl)oxy)quinolin-7-yl)morp holine

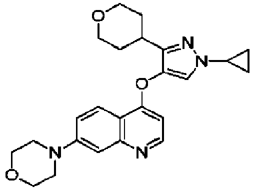

The preparation method was the same as Example 1, except that morpholine was used instead of 2-(4-amino-2-pyridinyl)propane-2-ol hydrochloride, to obtain the title compound. LC-MS m/z: 420.9[M+H]⁺. ¹HNMR(400 MHz, CDCl₃) δ8.55(d, *J*=5.2 Hz, 1H), 8.16(d, *J*=9.2 Hz, 1H), 7.35(s, 2H), 7.30(dd, *J*=9.2, 2.1 MHz, 1H), 6.47(d, *J*=5.3 Hz, 1H), 3.97-3.83(m, 5H), 3.56(td, *J*=10.9, 7.2, 3.7 Hz, 1H), 3.35-3.31(m, 5H), 2.91-2.61(m, 3H), 1.86(td, *J*=16.0, 12.5, 4.3 Hz, 2H), 1.73(d, *J*=11.7 Hz, 2H), 1.14-1.06(m, 2H), 1.04-0.96(m, 2H).

### Example 3 4-((1-cyclopropyl-3-(tetrahydro-2H-pyran-4-yl)-1H-pyrazol-4-yl)oxy)-7-(3-(trifluoromethyl) -5,6-dihydro-[1,2,4]triazolo[4,3-a]pyrazin-7(8H)-yl)quinoline

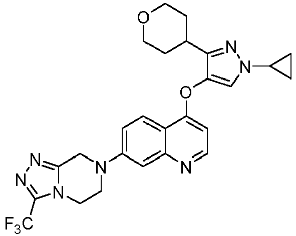

In a reaction flask, 7-bromo-4-((1-cyclopropyl-3-(tetrahydro-2*H*-pyran-4-yl)-1*H*-pyrazol-4-yl)oxy)quinoline (50 mg, 0.12 mmol), 3-(trifluoromethyl)-5,6,7,8-tetrahydro-[1,2,4]triazolo[4,3-*a*]pyrazine hydrochloride (35 mg, 0.14 mmol), cesium carbonate (118 mg, 0.36 mmol), 2-(dicyclohexylphosphino)-2,4,6-triisopropylbiphenyl (14 mg, 0.024 mmol) and palladium acetate (3 mg, 0.012 mmol) were placed, 5 mL of dioxane was added, to react under the protection of nitrogen for 3 h at 100°C with stirring. After the reaction was completed, the resulting product was allowed to return to room temperature, diluted with water, and then extracted with ethyl acetate. The organic phase was washed with water and a saturated aqueous solution of sodium chloride, dried with anhydrous sodium sulfate, concentrated under reduced pressure, and purified by column chromatography to obtain the title compound. LC-MS m/z: 526.3[M+H]⁺. ¹HNMR(400 MHz, CDCl₃) δ: 8.63(d, *J*=5.2 Hz, 1H), 8.29(d, *J*=7.1 MHz, 1H), 7.52(s, 1H), 7.45-7.30(m, 2H), 6.57(d, *J*=5.3 Hz, 1H), 4.85(s, 2H), 4.37(t, *J*=5.2 Hz, 2H), 3.99-3.94(m, 2H), 3.91(s, 2H), 3.59(m, 1H), 3.36(m, 2H), 2.88-2.75(m, 1H), 1.75(d, *J*=13.6 Hz, 4H), 1.17-1.10(m, 2H), 1.05(m, 2H). ¹HNMR(400 MHz, DMSO) δ8.59(d, *J*=5.2 Hz, 1H), 8.17(d, *J*=11.2 Hz, 1H), 7.94(s, 1H), 7.64(dd, *J*=11.2, 2.4 Hz, 1H), 7.45(d, *J*=2.4 Hz, 1H), 6.56(d, *J*=5.2 Hz, 1H), 4.91(s, 2H), 4.34(t, *J*=5.1 MHz, 2H), 4.02(t, *J*=5.2 Hz, 2H), 3.88-3.71(m, 2H), 3.73-3.63(m, 1H), 3.30-3.19(m, 2H), 2.82-2.65(m, 1H), 1.78-1.59(m, 4H), 1.12-1.01(m, 2H), 1.01-0.88(m, 2H).

### Example 4 4-(4-((1-cyclopropyl-3-(tetrahydro-2H-pyran-4-yl)-1H-pyrazol-4-yl)oxy)quinolin-7-yl)thiom orpholine-1,1-dioxide

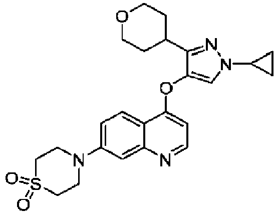

The preparation method was the same as Example 1, except that thiomorpholine-1,1-dioxide was used instead of 2-(4-amino-2-pyridinyl)propane-2-ol hydrochloride, to obtain the title compound. LC-MS m/z: 469.2[M+H]⁺. ¹HNMR(400 MHz, DMSO) δ8.57(d, *J*=5.2 Hz, 1H), 8.14(d, *J*=9.2 Hz, 1H), 7.93(s, 1H), 7.54(dd, 1H), 7.39(d, *J*=2.3 Hz, 1H), 6.54(d, *J*=5.2 Hz, 1H), 3.99(s, 4H), 3.77(dd, *J*=8.1, 3.0 Hz, 2H), 3.69(m, 1H), 3.29-3.24(m, 2H), 3.20(s, 4H), 2.79-2.68(m, 1H), 1.72-1.59(m, 4H), 1.09-1.02(m, 2H), 0.95(dt, *J*=12.6,6.1 MHz, 2H).

### Example 5 1-(4-((1-cyclopropyl-3-(tetrahydro-2H-pyran-4-yl)-1H-pyrazol-4-yl)oxy)quinolin-7-yl)-4-me thylpiperidin-4-ol

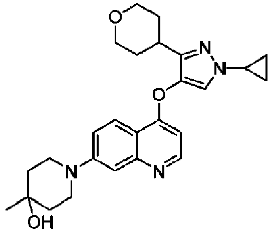

The preparation method was the same as Example 1, except that 4-methyl-4-hydroxypiperidine was used instead of 2-(4-amino-2-pyridinyl)propane-2-ol hydrochloride, to obtain the title compound. LC-MS m/z: 449.2[M+H]⁺. ¹HNMR(400 MHz, DMSO) δ8.51(d, *J*=5.3 Hz, 1H), 8.06(d, *J*=9.3 Hz, 1H), 7.92(s, 1H), 7.47(dd, *J*=9.4,2.4 Hz, 1H), 7.20(d, *J*=2.3 Hz, 1H), 6.47(d, *J*=5.3 Hz, 1H), 4.38(s, 1H), 3.84-3.73(m, 2H), 3.68(m, 1H), 3.63-3.50(m, 2H), 3.25(m, 4H), 2.73(m, 1H), 1.63(m,8H), 1.17(s, 3H), 1.09-1.01(m, 2H), 0.94(td, *J*=7.4, 5.5 Hz, 2H).

### Example 6 4-((1-cyclopropyl-3-(tetrahydro-2H-pyran-4-yl)-1H-pyrazol-4-yl)oxy)-7-(2-(2,2,2-trifluoroet hyl)-2,6-dihydropyrrolo[3,4-c]pyrazole-5(4H)-yl)quinoline

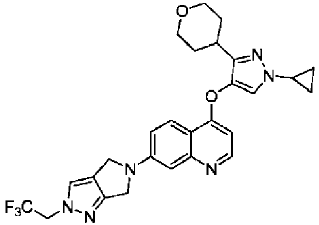

The preparation method was the same as Example 3, except that 2-(2,2,2-trifluoroethyl)-2,4,5,6-tetrahydropyrrolo[3,4-c]pyrazole hydrochloride was used instead of 3-(trifluoromethyl)-5,6,7,8-tetrahydro-[1,2,4]triazolo[4,3-*a*]pyrazine hydrochloride, to obtain the title compound. LC-MS m/z: 525.2[M+H]⁺. ¹HNMR(400 MHz, CDCl₃) δ8.57-8.53(m, 1H), 8.22(d, *J*=9.0 Hz, 1H), 7.47(s, 1H), 7.40-7.34(m, 1H), 7.13-7.03(m, 2H), 6.42(t, *J*=5.0 Hz, 1H), 4.78-4.70(m, 2H), 4.65(d, *J*=15.7 Hz, 2H), 4.61(s, 2H), 3.97-3.87(m, 2H), 3.65-3.53(m, 1H), 3.41-3.32(m, 2H), 2.89-2.76(m, 1H), 1.97-1.82(m, 2H), 1.80-1.71(m, 2H), 1.17-1.10(m, 2H), 1.07-1.00(m, 2H).

### Example 7 4-((1-cyclopropyl-3-(tetrahydro-2H-pyran-4-yl)-1H-pyrazol-4-yl)oxy)-7-(1-methyl-1H-pyra zol-4-yl)quinoline

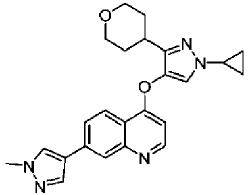

7-bromo-4-((1-cyclopropyl-3-(tetrahydro-2*H*-pyran-4-yl)-1*H*-pyrazol-4-yl)oxy)qui noline (650 mg, 1.57 mmol), 1-methyl-1*H*-pyrazole-4-boronic acid pinacol ester (650 mg, 3.15 mmol), potassium carbonate (430 mg, 3.15 mmol) and 1,1'-bisdiphenylphosphinoferrocene dichloropalladium (55 mg, 0.08 mmol) were placed in a reaction flask, dioxane (20 mL) and water (2 mL) were added, and heated up to 100°C to react under the protection of nitrogen for 1.5 h with stirring. After the reaction was completed, water and ethyl acetate were added to extract. The organic phase was washed with water and saturated aqueous solution of sodium chloride, dried with anhydrous sodium sulfate, concentrated under reduced pressure, and purified by column chromatography to obtain the title compound. LC-MS m/z: 416.2[M+H]⁺. ¹HNMR(400 MHz, CDCl₃) δ8.67(d, *J*=4.9Hz, 1H), 8.30(d, *J*=8.5 Hz, 1H), 8.16(s, 1H), 7.94(s, 1H), 7.80(s, 1H), 7.72(d, *J*=8.5 Hz, 1H), 7.39(s, 1H), 6.61(d, *J*=5.0 Hz, 1H), 4.00(s,3H), 3.93(d, *J*=10.3 Hz, 2H), 3.59(d, *J*=3.5 Hz, 1H), 3.36(t, *J*=11.4 Hz, 2H), 2.82(t, *J*=11.6 Hz, 1H), 1.94-1.83(m, 2H), 1.77(d, *J*=12.6 Hz, 2H), 1.12(m, 2H), 1.04(m, 2H).

### Example 8 4-((1-cyclopropyl-3-(tetrahydro-2H-pyran-4-yl)-1H-pyrazol-4-yl)oxy)-7-(1H-pyrazol-4-yl)q uinoline

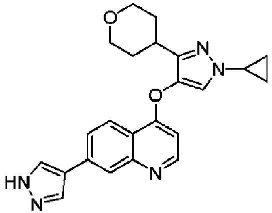

The preparation method was the same as Example 7, except that 4-(4,4,5,5-tetramethyl-1,3,2-dioxaborolan-2-yl)-1*H*-pyrazole was used instead of 1-methyl-1*H*-pyrazole-4-boronic acid pinacol ester, to obtain the title compound. LC-MS m/z: 402.2[M+H]⁺. ¹HNMR(400 MHz, DMSO) 813.11(s, 1H), 8.68(d, *J*=5.2 Hz, 1H), 8.44(s, 1H), 8.26(d, *J*=8.8 Hz, 2H), 7.98(s, 1H), 7.96(dd, *J*=8.6,1.7 Hz, 1H), 6.70(d, *J*=5.2 Hz, 1H), 5.76(s, 1H), 3.78(dt, *J*=11.2, 3.1 MHz, 2H), 3.70(m, 1H), 3.27(m, 2H), 2.81-2.71(m, 1H), 1.68(dt, *J*=13.6, 6.8 Hz, 4H), 1.11-1.03(m, 2H), 0.96(td, *J*=7.5, 5.5 Hz, 2H).

### Example 9 4-((1-cyclopropyl-3-(tetrahydro-2H-pyran-4-yl)-1H-pyrazol-4-yl)oxy)-7-(1-(methylsulfonyl) -1H-pyrazol-4-yl)quinoline

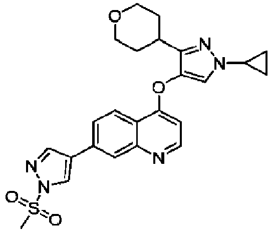

4-((1-cyclopropyl-3-(tetrahydro-2*H*-pyran-4-yl)-1*H*-pyrazol-4-yl)oxy)-7-(1*H*-pyraz ol-4-yl)quinoline (50 mg, 0.12 mmol) was dissolved in dichloromethane to which triethylamine was added (18 mg, 0.18 mmol) and methanesulfonyl chloride (17 mg, 0.14 mmol) was added in ice bath, then they were stirred for 2 h at room temperature. After the reaction was completed, methanol was added to quench the reaction, and water and ethyl acetate were added to extract. The organic phase was washed with water and saturated brine, dried with anhydrous sodium sulfate, concentrated under reduced pressure, and purified by thin layer chromatography to obtain the title compound. LC-MS m/z: 480.2[M+H]⁺. ¹HNMR(400 MHz, CDCl₃) δ8.72(d, *J*=5.1 MHz, 1H), 8.43(s, 1H), 8.37(d, *J*=8.6 Hz, 1H), 8.26(d, *J*=10.4 Hz, 2H), 7.73(dd, *J*=8.6,1.5 Hz, 1H), 7.41(s, 1H), 6.68(d, *J*=S.2 Hz, 1H), 3.98-3.88(m, 2H), 3.60(m, 1H), 3.43(s,3H), 3.37(td, *J*=11.7,1.9Hz, 2H), 2.83(m, 1H), 1.95-1.83(m, 2H), 1.82-1.73(m, 2H), 1.17-1.13(m, 2H), 1.05(m, 2H).

### Example 10 4-((1-cyclopropyl-3-(tetrahydro-2H-pyran-4-yl)-1H-pyrazol-4-yl)oxy)-7-(1-(2-(methylsulfon yl)ethyl)-1H-pyrazol-4-yl)quinoline

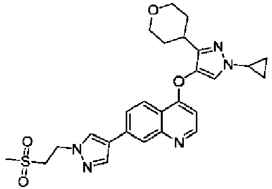

To a reaction flask, 4-((1-cyclopropyl-3-(tetrahydro-2*H*-pyran-4-yl)-1*H*-pyrazol-4-yl)oxy)-7-(1*H*-pyrazol-4-yl)q uinoline (81 mg, 0.2 mmol), methylvinylsulfone (22 µL) and potassium carbonate (56 mg, 0.4 mmol) were added, and dissolved in *N*,*N*-dimethylformamide (1 mL), to react for 2 h at 80°C with stirring. After the reaction was completed, water and ethyl acetate were added to extract. The organic phase was dried with anhydrous sodium sulfate, concentrated under reduced pressure, and purified by column chromatography to obtain the title compound. LC-MS m/z: 508.3[M+H]⁺. ¹HNMR(400 MHz, CDCl₃) 88.71(d, *J*=5.2 Hz, 1H), 8.35(d, *J*=8.6 Hz, 1H), 8.21(s, 1H), 8.06(s, 1H), 7.99(s, 1H), 7.73(dd, *J*=8.6, 1.7 Hz, 1H), 7.43(s, 1H), 6.66(d, *J*=5.2 Hz, 1H), 4.73(t,2H), 4.00-3.89(m, 2H), 3.74(t, *J*=6.0 Hz, 2H), 3.67-3.58(m, 1H), 3.45-3.33(m, 2H), 2.91-2.79(m, 1H), 2.60(s, 3H), 1.98-1.85(m, 2H), 1.82-1.74(m, 2H), 1.19-1.13(m, 2H), 1.10-1.03(m, 2H).

### Example 11 1-(4-(4-((1-cyclopropyl-3-(tetrahydro-2H-pyran-4-yl)-1H-pyrazol-4-yl)oxy)quinolin-7-yl)-1 H-pyrazol-1-yl)-2-methylpropan-2-ol

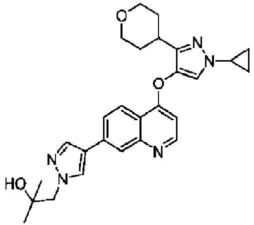

4-((1-cyclopropyl-3-(tetrahydro-2*H*-pyran-4-yl)-1*H*-pyrazol-4-yl)oxy)-7-(1*H*-pyraz ol-4-yl)quinoline (40 mg, 0.1 mmol), 2,2-dimethyloxirane (2 mL) and cesium carbonate (48 mg, 0.15 mmol) were added to a microwave tube, to react under microwave for 30 min at 120°C with stirring. After the reaction was finished, the reaction solution was diluted with dichloromethane, and filtered. The filtrate was collected, concentrated under reduced pressure, and purified by thin layer chromatography to obtain the title compound. LC-MS m/z: 474.2[M+H]⁺. ¹HNMR(400 MHz, DMSO) δ8.67(d, *J*=5.2 Hz, 1H), 8.33(s, 1H), 8.26(d, *J*=8.7 Hz, 1H), 8.19(d, *J*=1.5 Hz, 1H), 8.11(s, 1H), 7.97(s, 1H), 7.91(dd, *J*=8.7,1.7 Hz, 1H), 6.69(d, *J*=5.2 Hz, 1H), 4.80(s, 1H), 4.08(s, 2H), 3.81-3.74(m, 2H), 3.69(m, 1H), 3.24(dd, *J*=9.3, 4.9 Hz, 2H), 2.80-2.69(m, 1H), 1.67(dt, *J*=13.8, 6.7 Hz, 4H), 1.12(s, 6H), 1.09-1.04(m, 2H), 0.97-0.92(m, 2H).

### Example 12 Preparation of 4-((1-cyclopropyl-3-(tetrahydro-2H-pyran-4-yl)-1H-pyrazol-4-yl)oxy)-7-(1-isopropyl-1H-pyra zol-4-yl)quinoline

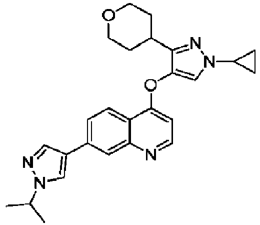

The preparation method was the same as Example 7, except that the commercial available raw material 1-isopropyl-1*H*-pyrazol-4-boronic acid pinacol ester was used instead of 1-methyl-1*H*-pyrazol-4-boronic acid pinacol ester, to obtain the title compound. LC-MS m/z: 444.2[M+H]⁺. ¹HNMR(400 MHz, DMSO) δ8.68(d, *J*=5.2 Hz, 1H), 8.50(s, 1H), 8.24(dd, *J*=13.9, 4.9Hz, 2H), 8.11(s, 1H), 7.97(s, 1H), 7.93(dd, *J*=8.7,1.5 Hz, 1H), 6.70(d, *J*=5.2 Hz, 1H), 4.54(dq,*J*=13.5,6.8 Hz, 1H), 3.78(dd, *J*=8.1, 3.0 Hz, 2H), 3.70(dq, *J*=11.1, 3.8 Hz, 1H), 3.30-3.21(m, 2H), 2.82-2.70(m, 1H), 1.66(dt, *J*=19.1, 6.8 Hz, 4H), 1.49(d, *J*=6.7 Hz,6H), 1.11-1.03(m, 2H), 0.96(m, 2H).

### Example 13 Preparation of 4-((1-cyclopropyl-3-(tetrahydro-2H-pyran-4-yl)-1H-pyrazol-4-yl)oxy)-7-(1-(2,2,2-trifluoroet hyl)-1H-pyrazol-4-yl)quinoline

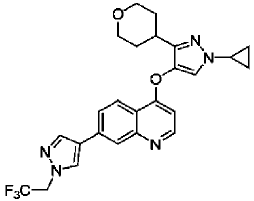

4-((1-cyclopropyl-3-(tetrahydro-2*H*-pyran-4-yl)-1*H*-pyrazol-4-yl)oxy)-7-(1*H*-pyraz ol-4-yl)quinoline (40 mg, 0.1 mmol) was dissolved in anhydrous tetrahydrofuran and stirred at 0°C, to which sodium hydride (5 mg, 0.15 mmol) was added to react for 1 h with stirring. 2,2,2-trifluoroethyl trifluoromethanesulfonate (46 mg, 0.2 mmol) was then added to react for 5 h at room temperature with stirring. Methanol was added to quench the reaction. Water and ethyl acetate were added to extract. The organic phase was washed with water and saturated brine, dried with anhydrous sodium sulfate, concentrated under reduced pressure, and purified by thin layer chromatography to obtain the title compound. LC-MS m/z: 484.2[M+H]⁺. ¹HNMR(400 MHz, CDCl₃) δ8.70(d, *J*=4.9Hz, 1H), 8.33(d, *J*=8.6 Hz, 1H), 8.21(s, 1H), 8.04(s, 1H), 7.93(s, 1H), 7.72(dd, *J*=8.6, 1.5 Hz, 1H), 7.40(s, 1H), 6.65(d, *J*=5.2 Hz, 1H), 4.80(q, *J*=8.3 Hz, 2H), 3.93(dd, *J*=11.5, 2.0 Hz, 2H), 3.60(m, 1H), 3.37(td, *J*=11.6, 1.8 Hz, 2H), 2.83(tt, *J*=11.6, 3.8 Hz, 1H), 1.90(m, 2H), 1.76(d, *J*=13.2 Hz, 2H), 1.18-1.10(m, 2H), 1.05(m, 2H).

### Example 14 4-((1-cyclopropyl-3-phenyl-1H-pyrazol-4-yl)oxy)-7-(1-methyl-1H-pyrazol-4-yl)quinoline

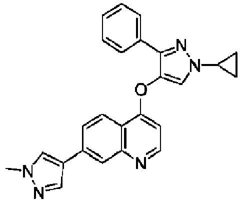

### Step 1: Preparation of 2-((7-bromoquinolin-4-yl)oxy)-1-phenylethan-1-one

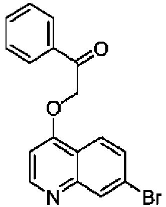

7-bromo-4-hydroxyquinoline (5 g, 22.3 mmol) and toluene (100 mL) were placed in a reaction flask, and 2-bromoacetophenone (8.8 g, 44.6 mmol) and silver carbonate (11 g, 44.6 mmol) were added. This reaction system was stirred to react at 100°C for 5 h and filtered to remove solids. The filtrate was concentrated under reduced pressure, and purified by column chromatography to obtain the title compound. LC-MS m/z: 342.2, 344.2[M+H]⁺.

### Step 2: Preparation of 2-((7-bromoquinolin-4-yl)oxy)-3-(dimethylamino)-1-phenylprop-2-en-1-one

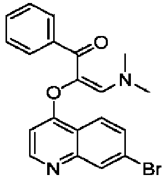

2-((7-bromoquinolin-4-yl)oxy)-1-phenylethan-1-one (1 g, 2.9 mmol) and *N*,*N-*dimethylformamide dimethyl acetal (10 mL) were placed in a reaction flask to react for 1 h at 100°C with stirring. After the reaction was finished, the resulting product was cooled down to room temperature, and concentrated under reduced pressure. The residue was dissolved in ethyl acetate, washed with water and a saturated aqueous solution of sodium chloride. The organic phase was dried with anhydrous sodium sulfate, and concentrated under reduced pressure to obtain the title product.

### Step 2: Preparation of 7-bromo-4-((3-phenyl-1H-pyrazol-4-yl)oxy)quinoline

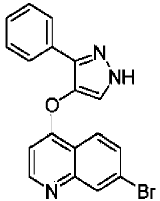

2-((7-bromoquinolin-4-yl)oxy)-3-(dimethylamino)-1-phenylprop-2-en-1-one (1 g, 2.52 mmol) were placed in a reaction flask to which acetic acid was added (10 mL), and cooled down to 0°C. Hydrazine hydrate (0.7 g) was added and stirred overnight under the condition of nitrogen at room temperature. The mixture was introduced into an ice-water mixture (25 mL), and extracted with ethyl acetate. The organic layer was combined, washed twice with water (50 mL), 0.5 M sodium hydrate and saturated brine successively, dried with anhydrous sodium sulfate. The resulting organic phase was concentrated to obtain the title compound. LC-MS m/z: 366.1, 368.1[M+H]⁺.

### Step 3: Preparation of 7-bromo-4-((1-cyclopropyl-3-phenyl-1H-pyrazol-4-yl)oxy)quinoline

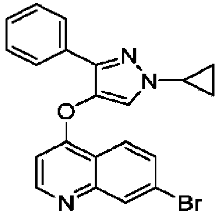

2,2-bipyridine (0.42 g, 2.7 mmol), copper acetate (0.5 g, 2.7 mmol) and 1,2-dichloroethane (10 mL) were placed in a reaction flask, refluxed at 75°C for 25 minutes, and then cooled down to room temperature. A 1,2-dichloroethane (10 mL) solution of 7-bromo-4-((3-phenyl-1*H*-pyrazol-4-yl)oxy)quinoline (0.9 g, 2.46 mmol) was added, and then cyclopropylboronic acid (0.42 g, 4.9 mmol) and sodium carbonate (0.5 g, 4.9 mmol) were added. The reaction mixture was stirred to react for 2 h at 75°C under an oxygen atmosphere, and cooled down to room temperature. The resulting product was diluted with ethyl acetate (100 mL), filtered through a short column packed with silica gel, and rinsed with ethyl acetate. The filtrate was washed with water and a saturated aqueous solution of sodium chloride, dried with anhydrous sodium sulfate. The resulting filtrate was concentrated, and purified by silica gel column chromatography to obtain the title compound. LC-MS m/z: 405.9, 407.9[M+H]⁺.

### Step 4: Preparation of 4-((1-cyclopropyl-3-phenyl-1H-pyrazol-4-yl)oxy)-7-(1-methyl-1H-pyrazol-4-yl)quinoline

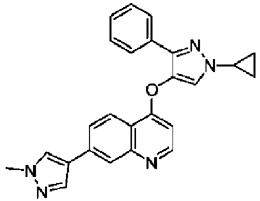

To a reaction flask, 7-bromo-4-((1-cyclopropyl-3-phenyl-1*H*-pyrazol-4-yl)oxy)quinoline (80 mg, 0.19 mmol), 1-methyl-1*H*-pyrazol-4-boronic acid pinacol ester (80 mg, 0.38 mmol), potassium carbonate (54 mg, 0.38 mmol) and 1,1'-bisdiphenylphosphinoferrocene dichloropalladium (7 mg, 0.009 mmol) were added, and 4 mL of dioxane and 0.5 mL of water were added to react under a nitrogen atmosphere at 80°C for 1 h with stirring. After the reaction was completed, water and then ethyl acetate were added to the reaction solution to extract. The organic phase was washed with water and saturated brine, dried with anhydrous sodium sulfate, concentrated under reduced pressure, and separated by thin layer chromatography to obtain the title compound. LC-MS m/z: 408.2[M+H]⁺. ¹HNMR(400 MHz, DMSO) 88.62(d, *J*=5.2 Hz, 1H), 8.41(s, 1H), 8.36(d, *J*=8.7 Hz, 1H), 8.19(s, 2H), 8.11(s, 1H), 7.94(dd, *J*=8.7, 1.5 Hz, 1H), 7.75-7.65(m, 2H), 7.29(t, *J*=7.4 Hz, 2H), 7.21(t, *J*=7.3 Hz, 1H), 6.72(d, *J*=S.2 Hz, 1H), 3.92(s, 3H), 3.89-3.78(m, 1H), 1.21-1.14(m, 2H), 1.08-0.99(m, 2H).

### Example 15 4-((1-cyclopropyl-3-(tetrahydro-2H-pyran-4-yl)-H-pyrazol-4-yl)oxy)-7-(4-(methylsulfonyl) phenyl)quinoline

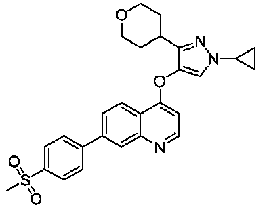

7-bromo-4-((1-cyclopropyl-3-(tetrahydro-2*H*-pyran-4-yl)-1*H*-pyrazol-4-yl)oxy)qui noline (50 mg, 0.121 mmol), 4-methanesulfonylphenylboronic acid (36 mg, 0.18 mmol), potassium carbonate (25 mg, 0.18 mmol) and 1,1'-bisdiphenylphosphinoferrocene dichloropalladium (5 mg, 0.006 mmol) were placed into a reaction flask to which dioxane (2 mL) and water (0.5 mL) was then added. Under the protection of nitrogen, they were heated up to 80°C to react for 2 h with stirring, and cooled down to room temperature. Water and ethyl acetate were added to extract. The organic phase was washed with water and a saturated aqueous solution of sodium chloride, dried with anhydrous sodium sulfate, concentrated under reduced pressure, and purified by column chromatography to obtain the title compound. LC-MS m/z: 490.2[M+H]⁺. ¹HNMR(400 MHz, DMSO) δ8.79(d, *J*=5.2 Hz, 1H), 8.47-8.38(m, 2H), 8.17(d, *J*=8.4 Hz, 2H), 8.12-8.04(m, 3H), 8.01(s, 1H), 6.84(d, *J*=5.2 Hz, 1H), 3.79(dd, *J*=8.3, 2.9Hz, 2H), 3.72(m, 1H), 3.31(s, 3H), 3.27(dd, *J*=7.1, 4.2 Hz, 2H), 2.84-2.72(m, 1H), 1.73-1.63(m, 4H), 1.11-1.05(m, 2H), 0.97(dt, *J*=12.7, 6.2 Hz, 2H).

### Example 16 2-(4-(4-((1-cyclopropyl-3-(tetrahydro-2H-pyran-4-yl)-1H-pyrazol-4-yl)oxy)quinolin-7-yl)py ridin-2-yl)propan-2-ol

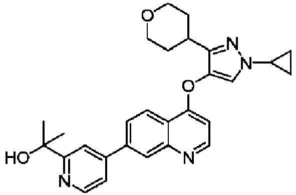

### Step 1: Preparation of 4-((1-cyclopropyl-3-(tetrahydro-2H-pyran-4-yl)-1H-pyrazol-4-yl)oxy)-7-(4,4,5,5-tetramethyl -1,3,2-dioxaborolan-2-yl)quinoline

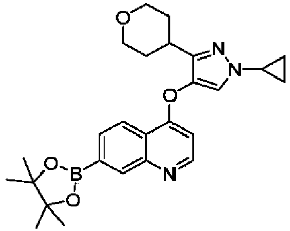

Into a reaction flask, 7-bromo-4-((1-cyclopropyl-3-(tetrahydro-2*H*-pyran-4-yl)-1*H*-pyrazol-4-yl)oxy)quinoline (110 mg, 0.27 mmol), bis(pinacolato)diboron (75 mg, 0.29 mmol), 1,1'-bisdiphenylphosphinoferrocene dichloropalladium (20 mg, 0.03 mmol) and potassium acetate (121 mg, 0.81 mmol) were placed, and 5 mL of dioxane was added. They were heated up to 100°C to react for 3 h with stirring. Water and ethyl acetate were added to extract. The organic phase was washed with water and a saturated sodium chloride solution, dried with anhydrous sodium sulfate, concentrated under reduced pressure to obtain the title compound which was used directly for the next step.

### Step 2: Synthesis of 2-(4-(4-((1-cyclopropyl-3-(tetrahydro-2H-pyran-4-yl)-1H-pyrazol-4-yl)oxy)quinolin-7-yl)py ridin-2-yl)propan-2-ol

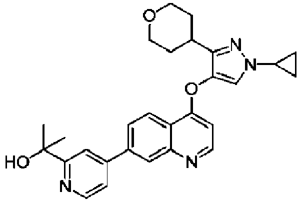

4-((1-cyclopropyl-3-(tetrahydro-2*H*-pyran-4-yl)-1*H*-pyrazol-4-yl)oxy)-7-(4,4,5,5-te tramethyl-1,3,2-dioxaborolan-2-yl)quinoline (120 mg, 0.26 mmol), 2-(4-bromopyridin-2-yl)-2-propanol (50 mg, 0.23 mmol) and 1,1'-bisdiphenylphosphinoferrocene dichloropalladium (15 mg, 0.026 mmol) were placed in a reaction flask, and dissolved in 5 mL of toluene by stirring at room room temperature with the atomosphere changed to argon gas. Sodium carbonate solution (0.5 mL, 2 M) was added. They were heated up to 80°C to react for 3 h with stirring, and then cooled down to room temperature. Ethyl acetate and water were added to extract. The organic phase was dried with anhydrous sodium sulfate, concentrated under reduced pressure, and purified by thin layer chromatography to obtain the title compound. LC-MS m/z: 470.9[M+H]⁺. ¹HNMR(400 MHz, DMSO) δ8.79(d, *J*=5.1 MHz, 1H), 8.63(d, *J*=4.9Hz, 1H), 8.51-8.38(m, 2H), 8.13(s, 1H), 8.08(d, *J*=8.7 Hz, 1H), 8.01(s, 1H), 7.75(d, *J*=4.0 Hz, 1H), 6.83(d, *J*=5.1 MHz, 1H), 5.40(s, 1H), 3.78(d, *J*=11.5 Hz, 2H), 3.74-3.67(m, 1H), 3.30-3.19(m, 2H), 2.79-2.73(m, 1H), 1.78-1.57(m, 4H), 1.52(s,6H), 1.08-1.03(m, 2H), 0.98-0.94(m, 2H).

### Example 17 Synthesis of 4-((1-cyclopropyl-3-(tetrahydro-2H-pyran-4-yl)-1H-pyrazol-4-yl)oxy)-7-methoxyquinoline-6-carboxamide

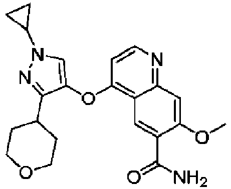

### Step 1: Preparation of 2-bromo-1-(tetrahydro-2H-pyran-4-yl)ethanone

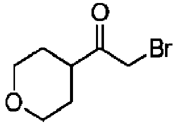

1-(tetrahydro-2*H*-pyran-4-yl)ethanone (10 g, 78.02 mmol) was added to 50 mL of methanol, and then cooled down to -10°C. Liquid bromine (4.0 mL, 78.02 mmol) was added under an argon atomosphere to the reaction. The reaction was performed for 45 min at 0°C, and subsequently for 45 min at 10°C. Sulfuric acid (27.5 mL, 11 M) was added to the above system, and stirred over night after the system returned to room temperature. After the reaction was finished, saturated brine and ethyl acetate were added to extract. The organic phase was combined, and washed with saturated sodium bicarbonate solution and water successivel, dried with anhydrous sodium sulfate, and concentrated under reduced pressure to obtain the title product.

### Step 2: Preparation of 2-oxo-2-(tetrahydro-2H-pyran-4-yl)ethyl benzoate

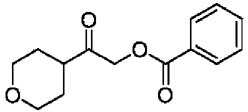

Benzoic acid (0.440 g, 3.6 mmol), potassium carbonate (0.829 g, 6.0 mmol) and anhydrous *N*,*N*-dimethylformamide (15 mL) were placed in a reaction flask. Under an argon atmosphere, anhydrous *N*,*N*-dimethylformamide solution of 2-bromo-1-(tetrahydro-2*H*-pyran-4-yl)ethanone (0.621 g, 3.0 mmol) was added to the above reaction system, to react overnight at room temperature. After the reaction was completed, saturated brine and ethyl acetate were added to extract. The organic phase was combined, washed with saturated brine, dried with anhydrous sodium sulfate, and concentrated under reduced pressure to obtain the title compound. LC-MS m/z: 249.2[M+H]⁺.

### Step 3: Preparation of 1-(dimethylamino)-3-oxo-3-(tetrahydro-2H-pyran-4-yl)prop-1-en-2-yl benzoate

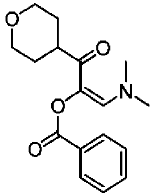

2-oxo-2-(tetrahydro-2*H*-pyran-4-yl)ethyl benzoate (0.745 g, 3.0 mmol) and 1,1-dimethoxy-*N*,*N*-dimethylmethylamine (1.54 mL, 12.75 mmol) were placed in a reaction flask to react at 90°C with stirring for 2 h. After the reaction was completed, the system was allowed to return to room temperature. The reaction solution was washed with ethyl acetate and saturated brine, dried with anhydrous sodium sulfate, and concentrated under reduced pressure to obtain the title compound. LC-MS m/z: 304.3[M+H]⁺.

### Step 4: Preparation of 3-(tetrahydro-2H-pyran-4-yl)-1H-pyrazol-4-yl methyl benzoate

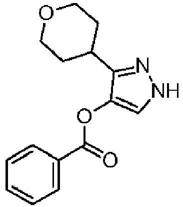

Under an argon atmosphere, hydrazine hydrate (1mL) was added at 0°C to an acetic acid (10 mL) solution of 1-(dimethylamino)-3-oxo-3-(tetrahydro-2*H*-pyran-4-yl)prop-1-en-2-yl benzoate (0.90 g, 2.97 mmol) and then heated up to room temperature to react overnight with stirring. After the reaction was completed, ethyl acetate and a saturated sodium bicarbonate solution was added to wash. The organic phase was dried with anhydrous sodium sulfate, and concentrated under reduced pressure to obtain the title product. LC-MS m/z: 273.2.

### Step 5: Preparation of 1-cyclopropyl-3-(tetrahydro-2H-pyran-4-yl)-1H-pyrazol-4-yl benzoate

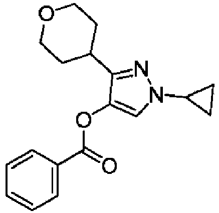

2,2'-bipyridine (0.515g, 3.3 mmol), copper acetate (0.599 g, 3.3 mmol) and 1, 2-dichloroethane (10 mL) were placed in a reaction flask to react at 75°C with stirring for 25 minutes, and then cooled down to room temperature. To the above reaction system, cyclopropylboronic acid (0.515 g, 6.0 mmol), sodium carbonate (0.636 g, 6.0 mmol) and a 1,2-dichloroethane (15 mL) solution of 3-(tetrahydro-2*H*-pyran-4-yl)-1*H*-pyrazol-4-yl methyl benzoate (0.670 g, 2.46 mmol) was added to react under an oxygen atmosphere at 75°C with stirring for 2 h. After the reaction was completed, the system was allowed to cool down to room temperature. Saturated brine and dichloromethane were added to extract. The organic phase was dried with anhydrous sodium sulfate, concentrated under reduced pressure, and purified by column chromatography to obtain the title compound. LC-MS m/z: =313.0[M+H]⁺.

### Step 6: Preparation of 1-cyclopropyl-3-(tetrahydro-2H-pyran-4-yl)-1H-pyrazol-4-ol

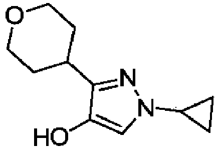

1-cyclopropyl-3-(tetrahydro-2*H*-pyran-4-yl)-1*H*-pyrazol-4-yl benzoate (0.50 g, 1.60 mmol) and 5.0 mLof ethanol were placed in a reaction flask. Then a sodium hydrate solution (1 M, 5.0 mL, 5.0 mmol) was added to the above reaction system, to react at room temperature for 3 h. After the reaction was completed, to the reaction solution, dilute hydrochloric acid was added to neutralize the system to neutral. The resulting product was extracted with dichloromethane, dried with anhydrous sodium sulfate, and concentrated under reduced pressure to obtain the title compound. LC-MS m/z: 209.1[M+H]⁺.

### Step 7: Preparation of 4-((1-cyclopropyl-3-(tetrahydro-2H-pyran-4-yl)-1H-pyrazol-4-yl)oxy)-7-methoxyquinoline-6-carboxamide

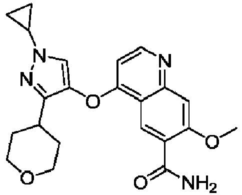

Raw materials, 1-cyclopropyl-3(-tetrahydro-2*H*-pyran-4-yl)-1*H*-pyrazol-4-ol (208 mg, 1 mmol), 4-chloro-7-methoxyquinoline-6-carboxamide (238 mg, 1 mmol) and potassium carbonate (280 mg, 2 mmol) were weighed and put into a 100 mL single mouth flask, to which 20 mL of *N*,*N*-dimethylformamide was then added, to react under the protection of argon gas at 130°C for 1 h. After extraction and column chromatography, the title product was obtained. LC-MS m/z: 408.9[M+H]⁺. ¹HNMR(400 MHz, DMSO) δ8.69(d, *J*=5.2 Hz, 1H), 8.66(s, 1H), 7.96(s, 1H), 7.88(s, 1H), 7.78(s, 1H), 7.52(s, 1H), 6.65(d, *J*=5.2 Hz, 1H), 3.78(d, *J*=11.2 Hz, 2H), 3.71(m, 1H), 3.36(s, 1H), 3.27(td, *J*=11.3,5.5 Hz, 2H), 2.83-2.69(m, 1H), 2.51(s, 2H), 1.73-1.58(m, 4H), 1.12-1.03(m, 2H), 0.96(dt, *J*=12.8, 6.2 Hz, 2H).

### Example 18 Preparation of 4-((1-cyclopropyl-3-(tetrahydro-2H-pyran-4-yl)-H-pyrazol-4-yl)oxy)-7-methoxy-N-(1-meth yl-1H-pyrazol-4-yl)quinoline-6-amine

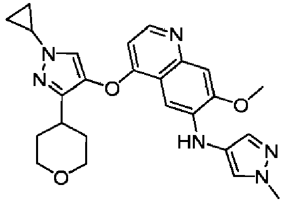

### Step 1: Preparation of 6-bromo-4-((1-cyclopropyl-3-(tetrahydro-2H-pyran-4-yl)-1H-pyrazol-4-yl)oxy)-7-methoxyq uinoline

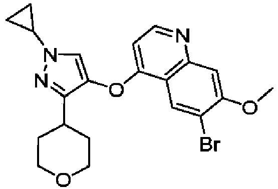

6-bromo-4-chloro-7-methoxyquinoline (27 mg, 0.1 mmol), 1-cyclopropyl-3-(tetrahydro-2*H*-pyran-4-yl)-1*H*-pyrazol-4-ol (21 mg, 0.1 mmol) and potassium carbonate (28 mg, 0.2 mmol) were weighed and put in a 50 mL single mouth flask, to which 10 mL of anhydrous *N*,*N-*dimethylformamide was added to react under the protection of argon gas at 140°C with stirring for 2 h. After the reaction was completed, 20 mL of water and ethyl acetate were added to extract. The organic phase was combined, washed with a saturated sodium chloride solution, dried with anhydrous sodium sulfate, and concentrated under reduced pressure to obtain the title compound which was directly used in the next reaction. LC-MS m/z: 444.3, 446.3 [M+H]⁺.

### Step 2: 4-((1-cyclopropyl-3-(tetrahydro-2H-pyran-4-yl)-1H-pyrazol-4-yl)oxy)-7-methoxy-N-(1-meth yl-1H-pyrazol-4-yl)quinoline-6-amine

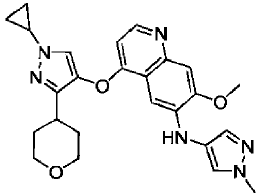

6-bromo-4-((1-cyclopropyl-3-(tetrahydro-2*H*-pyran-4-yl)-1*H*-pyrazol-4-yl)oxy)-7-methoxyquinoline (90 mg, 0.203 mmol), 1-methyl-1*H*-pyrazol-4-amine (19.7 mg, 0.203 mmol), tris(dibenzylideneacetone)dipalladium (9.2 mg, 0.0102 mmol), 4,5-bis(diphenylphosphino)-9,9-dimethylxanthene (11.75 mg, 0.0203 mmol) and cesium carbonate (99 mg, 0.305 mmol) were added to 15 mL of 1,4-dioxane, to react under the protection of argon gas at 100°C with stirring for 12 h. After the reaction was finished, filtration was carried out. The filtrate was concentrated under reduced pressure, and purified by column chromatography to obtain the title product. LC-MS m/z: 461.3[M+H]⁺. ¹HNMR(400 MHz, DMSO) δ8.34(d, *J*=5.2 Hz, 1H), 7.90(s, 1H), 7.70(s, 1H), 7.49(s, 1H), 7.43(s, 1H), 7.33(s, 1H), 7.28(s, 1H), 6.57(d, *J*=5.2 Hz, 1H), 4.03(s, 3H), 3.83(s, 3H), 3.78(dt, *J*=4.2, 3.1 MHz, 2H), 3.67(m, 1H), 3.25(dd, *J*=16.5, 9.1 MHz, 2H), 2.70(dt, *J*=15.5, 7.9 Hz, 1H), 1.69-1.58(m, 4H), 1.09-1.01(m, 2H), 0.94(dt, *J*=10.9, 5.3 Hz, 2H).

### Example 19 Synthesis of 4-((1-cyclopropyl-3-(tetrahydro-2H-pyran-4-yl)-1H-pyrazol-4-yl)oxa)-7-methoxy-N-(1-(oxet an-3-yl)-1H-pyrazol-4-yl)quinoline-6-amine

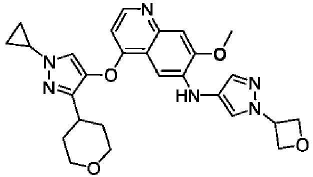

### Step 1: Preparation of 4-nitro-1-(oxetan-3-yl)-1H-pyrazole

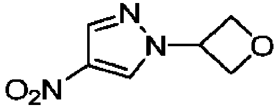

4-nitro-1-(oxetan-3-yl)-1*H*-pyrazole (1.13 g, 10 mmol), 3-iodooxetane (2.76 g, 15 mmol), cesium carbonate (6.52 g, 20 mmol) and anhydrous *N,N-*dimethylformamide (40 mL) were placed in a reaction flask, and under an argon gas atmosphere heated up to 100°C to react for 1 h with stirring. After the reaction was finished, filtration was carried out. Ethyl acetate and water were added to separate out the organic phase which was then concentrated under reduced pressure, and purified by column chromatography to obtain the title compound. LC-MS m/z: 170.1 [M+H]⁺.

### Step 2: Preparation of 1-(oxetan-3-yl)-1H-pyrazol-4-amine

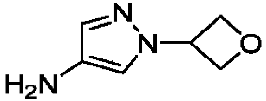

2-methyl-2-(4-nitro-1*H*-pyrazol-1-yl)propionamide (980 mg, 5.80 mmol) was weighed and dissolved in 30 mL of methanol. 160 mg of 10% palladium on carbon was added to react under a hydrogen gas atmosphere with stirring for 5 h. After filtration, the filtrate was concentrated under reduced pressure to obtain the title product. LC-MS m/z: 140. 1 [M+H]⁺.

### Step 3: Synthesis of 4-((1-cyclopropyl-3-(tetrahydro-2H-pyran-4-yl)-1H-pyrazol-4-yl)oxa)-7-methoxy-N-(1-(oxet an-3-yl)-1H-pyrazol-4-yl)quinoline-6-amine

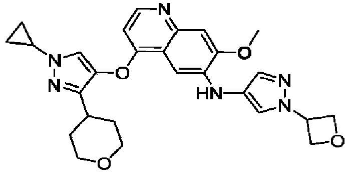

6-bromo-4-((1-cyclopropyl-3-(tetrahydro-2*H*-pyran-4-yl)-1*H*-pyrazol-4-yl)oxa)-7-methoxyquinoline (50 mg, 0.11 mmol), 1-(oxatan-3-yl)-1*H*-pyrazol-4-amine (10 mg, 0.248 mmol), tris(dibenzylideneacetone)dipalladium (10 mg, 0.011 mmol), 4,5-bis(diphenylphosphino)-9,9-dimethylxanthene (19 mg, 0.033 mmol) and cesium carbonate (40 mg, 0.23 mmol) were weighed and placed in a reaction flask, to which 10 mL of dioxane was then added, to react overnight under the protection of argon gas at 100°C. A filtrate was obtained after filtration, to which water and ethyl acetate were added to extract. The organic phase was concentrated. The resulting crude product was purified by column chromatography to obtain the title compound. LC-MS m/z: 503.4[M+H]⁺. ¹HNMR(400 MHz, DMSO) δ8.35(d, *J*=5.2 Hz, 1H), 7.90(s, 2H), 7.68(s, 1H), 7.53(s, 1H), 7.33(d, *J*=11.2 Hz, 2H), 6.58(d, *J*=5.2 Hz, 1H), 5.59(dd, *J*=14.0, 6.7 Hz, 1H), 4.97-4.85(m, 4H), 4.04(s,3H), 3.77(dt, *J*=11.1, 3.0 Hz, 2H), 3.67(m, 1H), 3.27-3.20(m, 2H), 2.70(dt, *J*=7.4, 6.0 Hz, 1H), 1.67-1.59(m, 4H), 1.07-1.02(m, 2H), 0.97-0.90(m, 2H).

### Example 20 4-(4-((1-cyclopropyl-3-(tetrahydro-2H-pyran-4-yl)-1H-pyrazol-4-yl)oxy)quinolin-7-yl)benze nesulfonamide

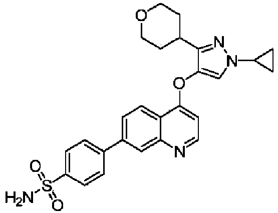

The preparation method was the same as Example 7, except that 4-(aminosulfonyl)phenylboronic acid was used instead of 1-methyl-1*H*-pyrazole-4-boronic acid pinacol ester, to obtain the title compound. LC-MS m/z: 491.1[M+H]⁺. ¹HNMR(400 MHz, CDCl₃) δ8.76(d, *J*=5.2 Hz, 1H), 8.43(d, *J*=8.7 Hz, 1H), 8.26(d, *J*=1.4 Hz, 1H), 8.05(d, *J*=8.5 Hz, 2H), 7.88-7.77(m, 3H), 7.43(s, 1H), 6.73(d, *J*=5.2 Hz, 1H), 5.36(s, 2H), 4.07-3.81(m, 2H), 3.65-3.56(m, 1H), 3.43-3.32(m, 2H), 2.91-2.75(m, 1H), 1.95-1.84(m, 2H), 1.79(d, *J*=1.9Hz, 2H), 1.19-1.12(m, 2H), 1.11-1.02(m, 2H).

### Example 21 4-((1-cyclopropyl-3-(tetrahydro-2H-pyran-4-yl)-1H-pyrazol-4-yl)oxy)-7-(6-(methylsulfonyl) pyridin-3-yl)quinoline

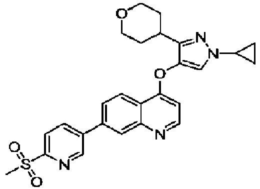

The preparation method was the same as Example 7, except that 2-methylsulfonyl pyridine-5-boronic acid was used instead of 1-methyl-1*H*-pyrazole-4-boronic acid pinacol ester, to obtain the title compound. LC-MS m/z: 491.1[M+H]⁺. ¹HNMR(400 MHz, CDCl₃) δ9.12(d, *J*=1.7 Hz, 1H), 8.79(d, *J*=5.2 Hz, 1H), 8.50(d, *J*=8.6 Hz, 1H), 8.37(d, *J*=1.5 Hz, 1H), 8.34-8.28(m, 1H), 8.27-8.22(m, 1H), 7.84(dd, *J*=8.6, 1.7 Hz, 1H), 7.44(s, 1H), 6.76(d, *J*=5.2 Hz, 1H), 3.94(dd, *J*=11.6,2.1 MHz, 2H), 3.66-3.58(m, 1H), 3.41-3.34(m, 2H), 3.32(s, 3H), 2.90-2.80(m, 1H), 1.95-1.86(m, 2H), 1.80-1.75(m, 2H), 1.19-1.13(m, 2H), 1.10-1.03(m, 2H).

### Example 22 4-((1-cyclopropyl-3-(tetrahydro-2H-pyran-4-yl)-1H-pyrazol-4-yl)oxy)-7-(1-(methylsulfonyl) -1,2,3,6-tetrahydropyridin-4-yl)quinoline

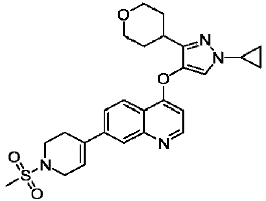

The preparation method was the same as Example 7, except that (1-(methylsulfonyl)-1,2,3,6-tetrahydropyridin-4-yl)boronic acid was used instead of 1-methyl-1*H*-pyrazole-4-boronic acid pinacol ester, to obtain the title compound. LC-MS m/z: 495.2[M+H]⁺. ¹HNMR(400 MHz, CDCl₃) δ8.68(d, *J*=5.2 Hz, 1H), 8.28(d, *J*=8.8 Hz, 1H), 8.03(d, *J*=1.3 Hz, 1H), 7.67(dd, *J*=8.8, 1.6 Hz, 1H), 7.39(s, 1H), 6.65(d, *J*=5.2 Hz, 1H), 6.34(s, 1H), 4.06(d, *J*=2.9Hz, 2H), 3.92(dd, *J*=11.6, 2.2 Hz, 2H), 3.63-3.56(m, 3H), 3.40-3.32(m, 2H), 2.89(s, 3H), 2.86-2.78(m, 3H), 1.94-1.84(m, 2H), 1.77-1.74(m, 2H), 1.17-1.11(m, 2H), 1.08-1.01(m, 2H).

### Example 23 4-((1-cyclopropyl-3-(tetrahydro-2H-pyran-4-yl)-1H-pyrazol-4-yl)oxy)-7-(2-(trifluoromethyl) -5,6-dihydro-[1,2,4]triazolo[1,5-a]pyrazin-7(8H)-yl)quinoline

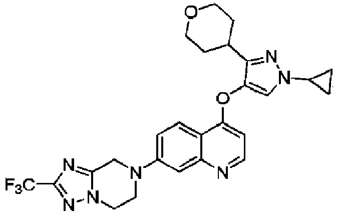

The preparation method was the same as Example 1, except that 2-(trifluoromethyl)-5,6,7,8-tetrahydro-[1,2,4]tiiazolo[1,5-*a*]pyrazine was used instead of 2-(4-amino-2-pyridinyl)propane-2-ol hydrochloride, to obtain the title compound. LC-MS m/z: 526.2[M+H]⁺. ¹HNMR(400 MHz, CDCl₃) δ8.63(d, *J*=5.2 Hz, 1H), 8.29(d, *J*=9.2 Hz, 1H), 7.48(d, *J*=2.3 Hz, 1H), 7.39(s, 1H), 7.36(dd, *J*=9.2, 2.4 Hz, 1H), 6.56(d, *J*=5.2 Hz, 1H), 4.75(s, 2H), 4.48(t, *J*=5.3 Hz, 2H), 4.02(t, *J*=5.4 Hz, 2H), 3.93(dd, *J*=11.6, 2.1 MHz, 2H), 3.65-3.53(m, 1H), 3.42-3.30(m, 2H), 2.88-2.75(m, 1H), 1.90-1.86(m, 2H), 1.79-1.71(m, 2H), 1.18-1.10(m, 2H), 1.10-1.00(m, 2H).

### Experimental Example 1 Evaluation on the in vitro activity of the compounds against ALK5 kinase

### 1. Experimental materials

### 1.1 Compounds

For the compounds of the present disclosure prepared in the above examples, each compound was prepared into 10 mM with DMSO, and then diluted to 3.333 µM, 1.111 µM, 370 nM, 123 nM, 41 nM, 14 nM, 4.6 nM, 1.5 nM and 0.5 nM successively.

### 1.2 Reagents and instruments

Reagents: ALK5, Cat.No.09-141, purchased from Carna; p38α, Cat. No.04-152, purchased from the Carna; TGFβR1 peptides, Cat.No.T36-58, purchased from SignalChem; dimethyl sulfoxide (DMSO), purchased from Sigma, US; EDTA, purchased from Sigma, US; and ADP-Glo Kinase Assay, Cat.No.v9102/3, 1×Kinase buffer (40 mM Tris, pH7.5, 0.10% BSA, 20 mM MgCl₂, 1 mM DTT), purchased from Promega, and prepared immediately before use.

Instrument: 2104 Multilabel Reader, purchased from Perkin Elmer, US.

### 2. Experimental methods

### 2.1 Preparation of 1× kinase buffer

1× assay buffer
40 mM Tris, pH7.5,
20 mM MgCl₂,
0.10% BSA
1 mM DTT

### 2.2 Compound preparation

### 2.2.1 Dilution of the compounds

2.2.1.1 Preparation of 50-fold compounds: The compounds were prepared into 500 µM, 50-fold concentration as the final concentration 10 µM for detecting the compounds, which was perfomed as following: into a second well of a 96-well plate, 95 µl of 100% DMSO was added, and then 5 µl of the 10 mM compound solution was added, to prepare a 1000 µM compound solution; into other wells, 60 µl of 100% DMSO was added; from the second well, 30 µl of the compound was drawn out and added into a third well; such 3-fold serial dilution was continued successively until 10 dilutions of concentration were made.

Diluting instruments: Automatic micropipette (Precision PRC384U).

### 2.2.1.2 Transferring 100 nl of the compound to a reaction plate using echo

### 2.3 Kinase reaction

### 2.3.1 Preparing a 2× kinase solution

The kinase was added to a 1x kinase buffer, to form a 2× enzyme solution. 2.5 µl of the 2× enzyme solution was added to a 384-well reaction plate which already contained 100 nl of 100% DMSO-dissolved compound, to incubate at room temperature for 10 minutes.

### 2.3.2 Preparing a 2× substrate solution

FAM labeled peptides and ATP were added to the 1× kinase buffer, to form a 2× substrate solution. 2.5 µl of the 2× substrate solution was added to the 384-well reaction plate.

### 2.4 Kinase reaction

The 384-well plate was incubated at 28°C for 120 minutes.

### 2.5 Detection of reaction results

2.5.1 An ADP-Glo reagent was equilibrated to room temperature.
2.5.2 5 µl of the reaction solution was transferred into a reaction well at another new 384-well plate.
2.5.3 5 µl of the ADP-Glo reagent was transferred into the reaction well at the 384-well plate to stop the reaction.
2.5.4 An incubation was performed at 28°C for 120 minutes.
2.5.5 10 µl of a kinase detection reagent was transferred into each reaction well, shaken for 1 minute, and was allowed to stand for 30 minutes at room temperature.

### 2.6 Data reading

The luminescence value of the sample was read from Envision.

### 2.7 Curve fitting

2.7.1 Luminescence reading data were copied from the Envision program.
2.7.2 The values of the luminescence reading were converted into the percent inhibition by the following formula.

Percent inhibition = (max - sample RLU) / (max - min)×100, where "min" stands for the fluorescence reading of the control sample with no enzyme added to the reaction; and "max" stands for the fluorescence reading of the sample with DMSO added as a control.

2.7.3 The date were imported into MS Excel and subjected to curve fitting using XLFit excel add-in version 5.4.0.8 and a fitting formula: Y= Bottom + (Top - Bottom) / (1 + (IC₅₀ / X)^HillSlope). The results are shown in Table 1.

**Table 1**

| Test compounds | IC₅₀ (nM) | | Test compounds | IC₅₀ (nM) | |
|---|---|---|---|---|---|
| | ALK5 (nM) | p38α (nM) | | ALK5 (nM) | p38α (nM) |
| Example 1 | 120 | 100000 | Example 2 | 98 | - |
| Example 3 | 80 | 100000 | Example 4 | 250 | - |
| Example 5 | 110 | - | Example 6 | 66 | - |
| Example 7 | 5.2 | 100000 | Example 8 | - | - |
| Example 15 | 3.2 | - | Example 16 | 100 | - |
| Example 17 | 66 | - | Example 18 | 6 | - |
| Example 19 | 10 | - | Example 20 | - | - |
| Example 21 | - | - | Example 22 | - | - |
| Example 23 | - | - | | | |

| | | | | | |
|---|---|---|---|---|---|
| "-" means no test data. | | | | | |

It can be seen from the above experimental results that the compounds of the present disclosure have good inhibitory activity on ALK5 kinase, and at same time have low inhibitory effect on p38α, thus have a high selectivity, demonstrating that the compounds of the present disclosure have lower side effects while producing higher efficacy.

### Experimental Example 2 Evaluation on the in vitro cell luciferase assay of the compounds

### 1. Experimental materials

Test compounds: For the compounds of the present disclosure prepared in the above examples, each compound was prepared into 4 mM using DMSO. Then, a 4-fold serial dilution was made to obtain 20000.00 nM, 5000.00 nM, 1250.00 nM, 312.5 nM, 78.125 nM, 19.53 nM, 4.88 nM and 1.22 nM successively.

Luc-Smad2/3-NIH3T3 mouse fibroblasts (engineered to overexpress SMAD2,3-responsive promoter) were provided by a laboratory of China Pharmaceutical University.

Reagents: DMEM, purchased from Invitrogen, US; FBS, purchased from Invitrogen, US; DMSO, purchased from Sigma, US; Glo Lysis Buffer, purchased from Progema, US; Bright-Glo Luciferase assay system, purchased from Promega, US; and TGFβ, purchased from PeproTech, US.

Instruments: MD SpectraMaX M3 multifunctional enzyme reader, purchased from Molecular Devices, US.

### 2. Experimental methods

### 2.1 Cell culture:

Cell recovery: The cells were dissolved by placing in a 3°C water bath, then transferred to a 15 mL of pre-warmed culture medium, and centrifuged at 1000 rpm for 5 minutes. After the culture medium was discarded, the cells were resuspend in 15 mL of fresh culture medium, transferred to a 10 cm dish, and placed in an incubator at 37° C with 5% CO₂ for culture. After 24 hours, the culture medium for the cells was changed with a fresh culture medium.

Cell passage: The recovered cells were transferred to a 50 mL sterile centrifuge tube and centrifuged at 1000 rpm for 5 minutes. The culture medium was discarded. The evenly dispersed cells was counted, and adjusted to an appropriate cell concentration of 15 mL of fresh culture medium, added to a 10 cm dish, and placed in an incubator at 37°C with 5% CO₂ for culture.

### 2.2 Experimental steps:

### Day 1: Cell-plating (at a transparent bottom 96-well plate)

Luc-Smad2/3-NIH3T3 cells were cultured normally in a 10 cm culture dish until the confluence reached 80%-90%. After digestion, they were collected in a 15 mL centrifuge tube, centrifuged at 1000 ×g for 5 minutes to remove the supernatant, and resuspended with 1 mL of medium, then diluted 10 times and counted. The cells was diluted according to the results from the count, and transferred into a 96-well plate at 4×10³/cells per well (100 µl of resuspended cells was added in each well).

### Day 2: drug treatment of cells

1-2 mg of the drug was weighed (weighed in advance), and prepared into a 4 mM stock solution using DMSO. After 24 hours, the culture medium was removed. The drug was diluted with 2% FBS culture medium. 100 µl of the 1× drug solution was added to make the final concentration of the drug to be 20000.00 nM, 5000.00 nM, 1250.00 nM, 312.5 nM, 78.125 nM, 19.53 nM, 4.88 nM, and 1.22 nM. TGFβ1 was present in each well at a final concentration of 4 ng/mL, and diluted with 2% FBS culture medium together with the compounds.

### Day 3: Fluorescence detection experiments

The Glo Lysis Buffer and the Bright-glo luciferase assay system as well as the cells were equilibrated to room temperature. After the cell supernatant was removed, the cells were dissolved evenly by adding 100 µl of the Glo Lysis Buffer to each well with shaking slowly, and lysed at room temperature for 5 minutes. After that, to each well, 100 µl of the Bright-glo luciferase assay system was added to incubate the cells for 5 minute at room temperature with shaking for 2 minutes. Then, 180 µl of the supernatant was transferred to a white bottom 96-well plate to detect chemiluminescence signal. The detection condition was 1 s.

2.3 Data processing: Graphpad Prism 5 software was used to perform nonlinear curve fitting and data analysis to obtain IC₅₀ by fitting. The experimental results are shown in Table 2.

**Table 2**

| Test compounds | IC₅₀ (nM) | Test compounds | IC₅₀ (nM) |
|---|---|---|---|
| | Luc-Smad2/3-NIH3T3 | | Luc-Smad2/3-NIH3T3 |
| Example 1 | 200 | Example 2 | 150 |
| Example 3 | 102 | Example 4 | 140 |
| Example 5 | 133 | Example 6 | 165 |
| Example 7 | 81 | Example 8 | 92 |
| Example 9 | 101 | Example 10 | 90 |
| Example 11 | 96 | Example 12 | 77 |
| Example 13 | 81 | Example 14 | 46 |
| Example 15 | 80 | Example 16 | 200 |
| Example 17 | 131 | Example 18 | 87 |
| Example 19 | 203 | Example 20 | 75 |
| Example 21 | 49 | Example 22 | 56 |
| Example 23 | 200 | | |

| | | | |
|---|---|---|---|
| "-" means no test data. | | | |

It can be seen from the above experiments that the compounds of the present disclosure all have good inhibitory activity on the TGFβ-ALK5-SMAD2/3 signaling pathway in NIH3T3 cells, and are very promising as therapeutic agents for various cancer-related diseases.

## Claims

1. A compound of general formula I, 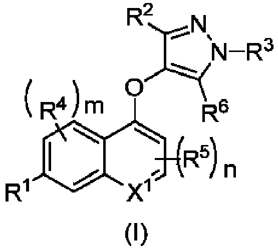 wherein
X¹ is selected from N and CH;
R¹ is selected from hydroxyl, cyano, carboxyl, nitro, alkyl, haloalkyl, hydroxyalkyl, alkoxyl, cycloalkyloxy, heterocycloalkyloxy, cycloalkylalkoxy, heterocyclylalkoxy, cycloalkylalkyl, heterocyclylalkyl, monoalkylamino, dialkylamino, cycloalkylamino, heterocyclylamino, arylamino, heteroarylamino, cycloalkyl, heterocyclyl, aryl, heteroaryl, aryl fused to heterocyclyl, and heteroaryl fused to heterocyclyl, which are optionally substituted with one or more halogen, hydroxyl, amino, carboxyl, cyano, nitro, oxo, alkylsulfonyl, aminosulfonyl, alkylsulfonylalkyl, alkyl, cycloalkyl, heterocyclyl, alkylheterocyclyl, alkoxyl, haloalkyl, hydroxyalkyl, aminoalkyl, carboxyalkyl, cyanoalkyl, nitroalkyl, cycloalkylalkyl, heterocycloalkylalkyl, alkoxyalkyl, monoalkylamino, dialkylamino, alkylacyl, alkoxyacyl, alkylacyloxy, aminoacyl, alkenylacyl, monoalkylaminoalkenylacyl, dialkylaminoalkenylacyl, monoalkylaminoacyl, dialkylaminoacyl, alkylacylamino or alkylacylaminoalkyl;
R², R³ are each independently selected from hydrogen, halogen, hydroxyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, alkenyl, alkynyl, cycloalkyl, heterocyclyl, aryl and heteroaryl, wherein the halogen, hydroxyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, alkenyl, alkynyl, cycloalkyl, heterocyclyl, aryl or heteroaryl are optionally substituted with one or more alkyl, haloalkyl, hydroxyl, hydroxyalkyl, halogen, oxo, alkoxyl, carboxyl, cyano, amino, monoalkylamino or dialkylamino;
R⁴ is selected from hydrogen, halogen, hydroxyl, alkyl, haloalkyl, hydroxyalkyl, alkoxyl, haloalkoxy, hydroxyalkoxy, nitro, carboxyl, cyano, amino, alkylamino, alkylacylamino, alkylacyl, aminoacyl, alkylaminoacyl, cycloalkyl, heterocycloalkyl, aryl, heteroaryl, cycloalkylamino, heterocycloalkylaryl, arylamino and heteroarylamino, wherein the halogen, hydroxyl, alkyl, haloalkyl, hydroxyalkyl, alkoxyl, haloalkoxy, hydroxyalkoxy, nitro, carboxyl, cyano, amino, alkylamino, alkylacylamino, alkylacyl, aminoacyl, alkylaminoacyl, cycloalkyl, heterocycloalkyl, aryl, heteroaryl, cycloalkylamino, heterocycloalkylaryl, arylamino and heteroarylamino are optionally substituted with one or more alkyl, alkoxyl, aryloxy, alkylamino, cycloalkyl, heterocycloalkyl, aryl, heteroaryl, arylamino, halogen, hydroxyl, amino, nitro, carboxyl, cyano, alkylacyl, aminoacyl, alkaminoacyl, sulfonyl or sulfhydryl;
R⁵ and R⁶ are each independently selected from hydrogen, halogen, hydroxyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino, and cycloalkyl; and
m and n are each independently selected from 1, 2 and 3,
or a pharmaceutically acceptable salt, an isomer, a solvate, a crystal or a prodrug thereof.

2. The compound or the isomer, pharmaceutically acceptable salt, crystal, solvate or prodrug thereof according to claim 1, wherein R¹ is selected from hydroxyl, cyano, carboxyl, nitro, C₁₋₆alkyl, haloC₁₋₆alkyl, hydroxyC₁₋₆alkyl, C₁₋₆alkoxyl, C₃₋₆cycloalkyloxy, C₃₋₆heterocycloalkyloxy, C₃₋₆cycloalkylC₁₋₃alkoxyl, C₃₋₆heterocyclylC₁₋₃alkoxyl, C₃₋₆cycloalkylC₁₋₃alkyl, C₃₋₆heterocyclylC₁₋₃alkyl, monoC₁₋₆alkylamino, diC₁₋₆alkylamino, C₃₋₆cycloalkylamino, C₃₋₆heterocyclylamino, C₆₋₁₂arylamino, C₅₋₈heteroarylamino, C₃₋₆cycloalkyl, C₃₋₆heterocyclyl, C₆₋₁₂aryl, C₅₋₁₂heteroaryl, C₆₋₁₂aryl fused to C₃₋₁₀heterocyclyl, and C₅₋₁₂heteroaryl fused to C₃₋₆heterocyclyl, which are optionally substituted with one or more halogen, hydroxyl, amino, carboxyl, cyano, nitro, oxo, C₁₋₆alkylsulfonyl, aminosulfonyl, C₁₋₆alkylsulfonylC₁₋₆alkyl, C₁₋₆alkyl, C₃₋₁₀cycloalkyl, C₃₋₁₀heterocyclyl, C₁₋₆alkylC₃₋₁₀heterocyclyl, C₁₋₆alkoxyl, haloC₁₋₆alkyl, hydroxyC₁₋₆alkyl, aminoC₁₋₆alkyl, carboxylC₁₋₆alkyl, cyanoC₁₋₆alkyl, nitroC₁₋₆alkyl, C₃₋₆cycloalkylC₁₋₆alkyl, C₃₋₆heterocycloalkylC₁₋₆alkyl, C₁₋₆alkoxyC₁₋₆alkyl, monoC₁₋₆alkylamino, diC₁₋₆alkylamino, C₁₋₆alkylacyl, C₁₋₆alkoxyacyl, C₁₋₆alkylacyloxy, aminoacyl, C₂₋₁₀alkenylacyl, monoC₁₋₆alkylaminoC₂₋₁₀alkenylacyl, diC₁₋₆alkylaminoC₂₋₁₀alkenylacyl, monoC₁₋₆alkylaminoacyl, diC₁₋₆alkylaminoacyl, C₁₋₆alkylacylamino or C₁₋₆alkylacylaminoC₁₋₆alkyl.

3. The compound or the isomer, pharmaceutically acceptable salt, crystal, solvate or prodrug thereof according to claim 1 or 2, wherein R² and R³ are each independently selected from hydrogen, fluorine, chlorine, bromine, iodine, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino, C₂₋₁₀alkenyl, C₂₋₁₀alkynyl, C₃₋₁₀cycloalkyl, 3- to 10-membered heterocycloalkyl, C₆₋₁₈aryl and 5- to 18-membered heteroaryl, wherein the fluorine, chlorine, bromine, iodine, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino, C₂₋₁₀alkenyl, C₂₋₁₀alkynyl, C₃₋₁₀cycloalkyl, 3- to 10-membered heterocycloalkyl, C₆₋₁₈aryl and 5- to 18-membered heteroaryl are optionally substituted with one or more alkyl, haloalkyl, hydroxyl, hydroxyalkyl, halogen, oxo, alkoxyl, carboxyl, cyano, amino, monoalkylamino or dialkylamino.

4. The compound or the isomer, pharmaceutically acceptable salt, crystal, solvate or prodrug thereof according to any one of claims 1-3, wherein R⁴ is selected from hydrogen, halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, C₁₋₆alkylamino, C₁₋₆alkylacylamino, C₁₋₆alkylacyl, aminoacyl, C₁₋₆alkylaminoacyl, C₃₋₈cycloalkyl, C₃₋₈heterocycloalkyl, C₆₋₁₂aryl, C₅₋₁₂heteroaryl, C₃₋₈cycloalkylamino, C₃₋₈heterocycloalkylaryl, C₆₋₁₂arylamino and C₅₋₈heteroarylamino, wherein the halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, C₁₋₆alkylamino, C₁₋₆alkylacylamino, C₁₋₆alkylacyl, aminoacyl, C₁₋₆alkylaminoacyl, C₃₋₈cycloalkyl, C₃₋₈heterocycloalkyl, C₆₋₁₂aryl, C₅₋₁₂heteroaryl, C₃₋₈cycloalkylamino, C₃₋₈heterocycloalkylaryl, C₆₋₁₂arylamino and C₅₋₈heteroarylamino are optionally substituted with one or more C₁₋₆alkyl, C₁₋₆alkoxyl, C₆₋₁₂aryloxy, C₁₋₆alkylamino, C₃₋₆cycloalkyl, C₃₋₆heterocycloalkyl, C₆₋₁₂aryl, C₅₋₈heteroaryl, C₆₋₁₂arylamino, halogen, hydroxyl, amino, nitro, carboxyl, cyano, alkylacyl, aminoacyl, alkaminoacyl, sulfonyl or sulfhydryl; and
R⁵ and R⁶ are each independently selected from hydrogen, halogen, hydroxyl, C₁₋₆alkyl, haloC₁₋₆alkyl, hydroxyC₁₋₆alkyl, C₁₋₆alkoxyl, haloC₁₋₆alkoxyl, hydroxyC₁₋₆alkoxyl, nitro, carboxyl, cyano, amino, monoC₁₋₆alkylamino, C₁₋₆alkylacylamino, C₁₋₆alkylacyl, aminoacyl, C₁₋₆alkylaminoacyl, diC₁₋₆alkylamino and C₃₋₁₀cycloalkyl.

5. The compound or the isomer, pharmaceutically acceptable salt, solvate, crystal or prodrug thereof according to any one of claims 1-4, wherein R¹ is selected from C₁₋₆alkoxyl, morpholinyl, piperidinyl, pyrazolyl, phenyl, pyridinyl, pyridineamino, pyrrolopyrazolyl, and triazolopyrazinyl, thiomorpholinyl, which are optionally substituted with one or more halogen, hydroxyl, amino, carboxyl, cyano, nitro, oxo, C₁₋₆alkylsulfonyl, aminosulfonyl, C₁₋₆alkylsulfonylC₁₋₆alkyl, C₁₋₆alkyl, C₃₋₁₀cycloalkyl, C₃₋₁₀heterocyclyl, C₁₋₆alkylC₃₋₁₀heterocyclyl, C₁₋₆alkoxyl, haloC₁₋₆alkyl, hydroxyC₁₋₆alkyl, aminoC₁₋₆alkyl, carboxylC₁₋₆alkyl, cyanoC₁₋₆alkyl, nitroC₁₋₆alkyl, C₃₋₆cycloalkylC₁₋₆alkyl, C₃₋₆heterocycloalkylC₁₋₆alkyl, C₁₋₆alkoxyC₁₋₆alkyl, monoC₁₋₆alkylamino, diC₁₋₆alkylamino, C₁₋₆alkylacyl, C₁₋₆alkoxyacyl, C₁₋₆alkylacyloxy, aminoacyl, C₂₋₁₀alkenylacyl, monoC₁₋₆alkylaminoC₂₋₁₀alkenylacyl, diC₁₋₆alkylaminoC₂₋₁₀alkenylacyl, monoC₁₋₆alkylaminoacyl, diC₁₋₆alkylaminoacyl, C₁₋₆alkylacylamino or C₁₋₆alkylacylaminoC₁₋₆alkyl;
R² and R³ are each independently selected from hydrogen, fluorine, chlorine, bromine, iodine, hydroxyl, methyl, ethyl, propyl, butyl, isopropyl, isobutyl, tertiary butyl, methylamino, ethylamino, propylamino, isopropylamino, cyclopropyl, cyclobutyl, cyclopentyl, cyclohexyl, phenyl, pyrrolyl, imidazolyl, pyrazolyl, thiazolyl, thienyl, furyl, pyridinyl, pyrazinyl, pyrimidyl, azetidinyl, oxetanyl, tetrahydropyrrolyl, tetrahydrofuranyl, piperidinyl, tetrahydropyranyl and morpholinyl, which are optionally substituted with one or more hydroxyl, methyl, ethyl, propyl, butyl, isopropyl, isobutyl, tertiary butyl, carboxyl, fluorine, chlorine, bromine, trifluoromethyl, trifluoroethyl, aminomethyl, aminoethyl, aminopropyl, methylamino, ethylamino, propylamino, isopropylamino, methoxy, ethoxy, propoxy, isopropoxy, oxo, formyl, acetyl, propanoyl, isopropionyl, vinyl, propenyl, ethynyl, propynyl, phenyl, naphthyl, pyrrolyl, imidazolyl, pyrazolyl, thiazolyl, thienyl, furyl, pyridinyl, pyrazinyl and pyrimidyl; and
R⁴ is selected from hydrogen, aminoacyl and pyrazolamino, wherein the aminoacyl and pyrazolamino are optionally substituted with one or more C₁₋₆alkyl, C₁₋₆alkoxyl, C₆₋₁₂aryloxy, C₁₋₆alkylamino, C₃₋₆cycloalkyl, C₃₋₆heterocycloalkyl, C₆₋₁₂aryl, C₅₋₈heteroaryl, C₆₋₁₂arylamino, halogen, hydroxyl, amino, nitro, carboxyl, cyano, alkylacyl, aminoacyl, alkaminoacyl, sulfonyl or sulfhydryl.

6. The compound or the isomer, pharmaceutically acceptable salt, solvate, crystal or prodrug thereof according to any one of claims 1 to 5, wherein general formula I has a structure of following general formula Ia, 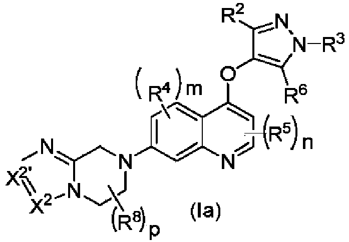 wherein R², R³, R⁴, R⁵, R⁶, m, and n are defined as in claims 1 to 5;
X² and X^{2'} are each independently selected from N and C(R⁷), wherein R⁷ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl;
R⁸ is selected from hydrogen, halogen, hydroxyl, oxo, alkylsulfonyl, alkylsulfonylalkyl, alkyl, haloalkyl, hydroxyalkyl, alkoxyl, haloalkoxy, hydroxyalkoxy, nitro, carboxyl, cyano, amino, monoalkylamino, alkylacylamino, alkylacyl, aminoacyl, alkylaminoacyl, dialkylamino and cycloalkyl; and
p is selected from 1, 2 and 3.

7. The compound or the pharmaceutically acceptable salt, isomer, solvate, crystal or prodrug thereof according to any one of claims 1 to 5, wherein general formula I has a structure of following general formula Id, 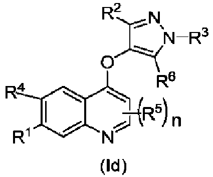 wherein R¹, R², R³, R⁴, R⁵, R⁶ and n are defined as in claims 1 to 5.

8. The compound or the isomer, pharmaceutically acceptable salt, crystal, solvate or prodrug thereof according to claim 1, wherein the compound is selected from the following compounds:

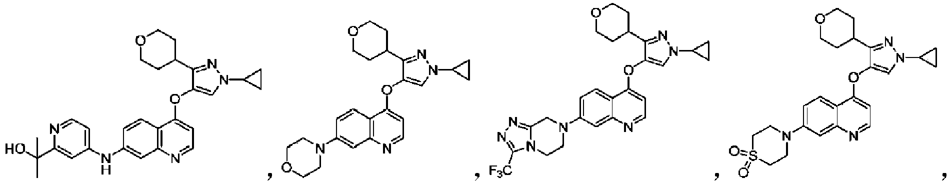

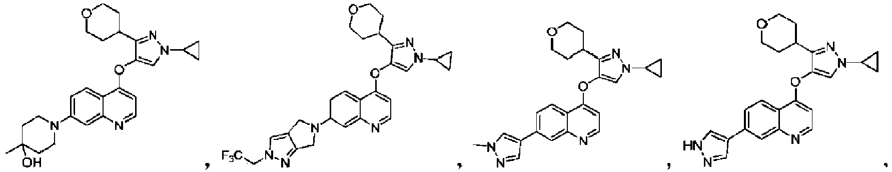

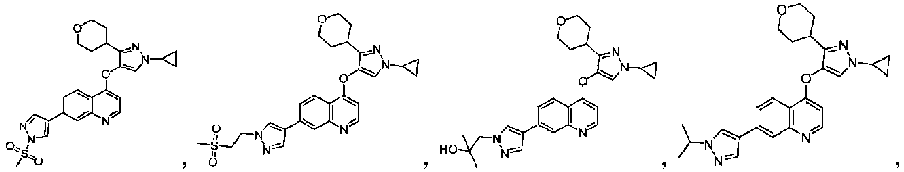

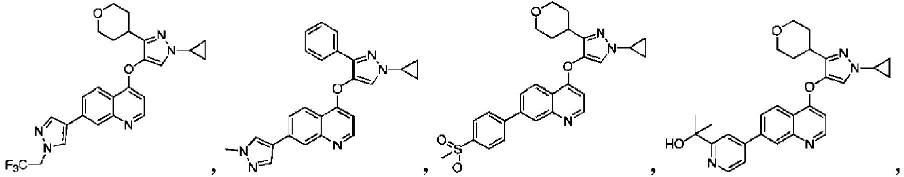

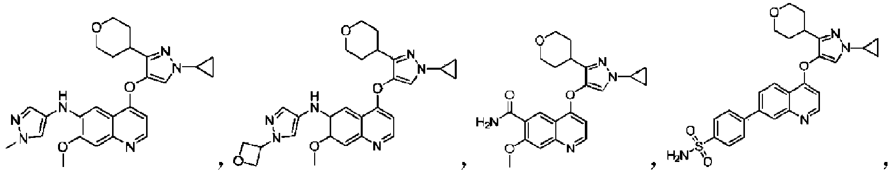

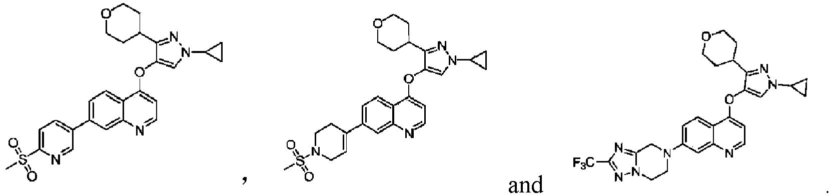

9. A pharmaceutical composition, comprising the compound or the pharmaceutically acceptable salt, isomer, solvate, prodrug thereof according to any one of claims 1 to 8 and a pharmaceutically acceptable carrier.

10. Use of the compound or the pharmaceutically acceptable salt, isomer, solvate or prodrug according to any one of claims 1-8 or the pharmaceutical composition according to claim 9 in the manufacture of a medicament for treating and/or preventing a disease related to TGF-βR1, preferably, the disease is a cancer, a tissue hyperplasia disease, a fibrotic or inflammatory disease.
